(19) Europäisches Patentamt
European Patent Office
Office européen des brevets

(11) **EP 4 585 162 A1**

(12) **EUROPEAN PATENT APPLICATION**

(43) Date of publication:
**16.07.2025 Bulletin 2025/29**

(21) Application number: **25152118.3**

(22) Date of filing: **15.01.2025**

(51) International Patent Classification (IPC):
**A61B 8/08** *(2006.01)* **A61B 8/00** *(2006.01)*
**G01S 7/52** *(2006.01)*

(52) Cooperative Patent Classification (CPC):
**A61B 8/485; A61B 8/08; A61B 8/463; A61B 8/469;
G01S 7/52022; G01S 7/52042; G01S 7/52071**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.01.2024 JP 2024004074
12.09.2024 JP 2024158291**

(71) Applicant: **Canon Medical Systems Corporation
Tochigi 324-0036 (JP)**

(72) Inventors:
• ITO, Daiki
Otawara-shi, 324-0036 (JP)
• IGARASHI, Yu
Otawara-shi, 324-0036 (JP)
• WATANABE, Masaki
Otawara-shi, 324-0036 (JP)
• HONJO, Yasunori
Otawara-shi, 324-0036 (JP)
• UTSUNOMIYA, Mio
Otawara-shi, 324-0036 (JP)

(74) Representative: **Marks & Clerk LLP
15 Fetter Lane
London EC4A 1BW (GB)**

(54) **ULTRASONIC DIAGNOSIS APPARATUS, METHOD, AND STORAGE MEDIUM**

(57) An ultrasound diagnosis apparatus (10) according to an embodiment includes: an analyzing unit (421), a setting unit (412), and an obtaining unit (411). The analyzing unit (421) is configured to analyze a shear wave that has propagated in a subject. The setting unit (412) is configured to set an obtaining condition for scan data on the basis of an analysis result of the shear wave. The obtaining unit (411) is configured to perform a scan on the subject on the basis of the obtaining condition and to obtain the scan data of the subject.

FIG.1

## Description

FIELD

Embodiments described herein relate generally to an ultrasound diagnosis apparatus, a method, and a storage medium.

BACKGROUND

In recent years, some ultrasound diagnosis apparatuses use Shear Wave Elastography (SWE) by which a firmness image is displayed by measuring a propagation velocity of a shear wave generated by a push pulse. For diffuse liver diseases, for example, SWE is one of useful techniques for evaluating firmness of a tissue in a non-invasive and quantitative manner.

SUMMARY OF INVENTION

An ultrasound diagnosis apparatus provided in an aspect of the present embodiment includes a first obtaining unit configured to obtain scan data by transmitting and receiving an ultrasound wave for observing a shear wave of a subject, a second obtaining unit configured, before the scan data is obtained, to obtain a biological signal of the subject, an analyzing unit configured to calculate an index for determining an obtaining condition for the scan data on the basis of the biological signal, and a setting unit configured to set the obtaining condition for the scan data on the basis of the index.
As the biological signal, the second obtaining unit may be configured to obtain a shear wave obtained by transmitting a push pulse and transmitting and receiving a tracking pulse.
The second obtaining unit may be configured to execute the transmission of the push pulse and the transmission/reception of the tracking pulse, by using an obtaining condition with which it is possible to obtain shear waves propagating in tissues having mutually-different tissue properties.
The analyzing unit may be configured to calculate a level of an elasticity value of the tissue of the subject, on the basis of the shear wave.
On the basis of the index, the setting unit may be configured to select the obtaining condition for the scan data from among a plurality of obtaining conditions set in advance.
The setting unit may be configured to obtain the obtaining condition for the scan data, by inputting the index to a model that outputs an obtaining condition for the scan data in response to an input of the index.
On the basis of the index, the setting unit may be configured to set at least one selected from between: a time period from when the biological signal is obtained to when the obtainment of the scan data is started; and a time period until obtainment of next scan data is started after the scan data is obtained.
A display controlling unit configured to exercise control so that a display unit displays the index may further be provided.
When the index does not satisfy a reference level, the display controlling unit may be configured to exercise control so as to display alert information related to the index.
When a plurality of pieces of scan data are to be consecutively obtained after the biological signal is obtained, the setting unit may be configured to set a transmission condition of a push pulse and a transmission/reception condition of a tracking pulse for observing the shear wave, on the basis of the index, and the first obtaining unit may be configured to obtain the plurality of pieces of scan data by using the transmission condition of the push pulse and the transmission/reception condition of the tracking pulse that were set.
A storage unit configured to store therein the obtaining condition for the scan data may further be provided, and the first obtaining unit may be configured to read the obtaining condition for the scan data from the storage unit and to further obtain the scan data on the basis of the read obtaining condition.
As the obtaining condition for the scan data, the setting unit may be configured to set at least one selected from among: a transmission condition of a push pulse and a transmission/reception condition of a tracking pulse for observing the shear wave; a condition related to a display based on the scan data; and a condition related to an analysis based on the scan data.
A method provided in an aspect of the present embodiment includes obtaining a biological signal of a subject, before scan data is obtained by transmitting and receiving an ultrasound wave for observing a shear wave of the subject, calculating an index for determining an obtaining condition for the scan data on the basis of the biological signal, setting the obtaining condition for the scan data on the basis of the index, and obtaining the scan data on the basis of the obtaining condition.
A storage medium provided in an aspect of the present embodiment storing therein, in a non-transitory manner, a program that causes a computer to execute processes of: obtaining a biological signal of a subject, before scan data is obtained by transmitting and receiving an ultrasound wave for observing a shear wave of the subject, calculating an index for determining an obtaining condition for the scan data on the basis of the biological signal, setting the obtaining condition for the scan data on the basis of the index, and obtaining the scan data on the basis of the obtaining condition.

An ultrasound diagnosis apparatus provided in an aspect of the present embodiment includes an analyzing unit configured to analyze a shear wave that has propagated in a subject, a setting unit configured to set an obtaining condition for scan data on the basis of an analysis result of the shear wave, and an obtaining unit configured to perform a scan on the subject on the basis of the obtaining condition and to obtain the scan data of the subject.

The analyzing unit may be configured to analyze a first shear wave that has propagated through various positions in a first measurement region of the subject, and on the basis of an analysis result of the first shear wave, the setting unit may be configured to set a transmission condition of a push pulse for generating a second shear wave in a second measurement region of the subject and a transmission/reception condition of a tracking pulse for observing the second shear wave, as the obtaining condition for the scan data.

The first measurement region may be smaller than the second measurement region.

On the basis of the analysis result of the first shear wave, the setting unit may be configured to set a condition including at least one of a position and a size of the second measurement region, as the obtaining condition for the scan data.

On the basis of the analysis result of the first shear wave, the setting unit may be configured to set, as the obtaining condition for the scan data, a condition including at least one selected from among: a measurement start time for measuring the second shear wave in various positions in the second measurement region; a measurement end time for the second shear wave; and a measurement time span.

The analyzing unit may be configured to obtain a tissue property index value indicating a tissue property of the subject in each of various positions in the first measurement region, as the analysis result of the shear wave, and the setting unit may be configured to set the obtaining condition for the scan data, on the basis of the tissue property index value in each of the various positions in the first measurement region.

The tissue property index value may be at least one selected from between: an elasticity index value indicating elasticity of a tissue of the subject; and a viscosity index value indicating viscosity of the tissue of the subject.

The analyzing unit may be configured to obtain a reliability index value indicating a reliability of a propagation of the first shear wave in each of various positions in the first measurement region, as the analysis result of the shear wave, and the setting unit may be configured to set the obtaining condition for the scan data on the basis of the reliability index value in each of the various positions in the first measurement region.

The setting unit may be configured to input the analysis result of the shear wave analyzed by the analyzing unit to a model that outputs an obtaining condition for the scan data in response to an input of the analysis result of the shear wave and to set the obtaining condition for the scan data output by the model.

A controlling unit configured to cause an ultrasound image to be displayed may be provided, the ultrasound image being based on the scan data of the subject obtained by the obtaining unit.

A controlling unit configured to cause an indicator to be displayed may be provided, the indicator indicating a position of the obtaining condition set by the setting unit within a range where it is possible to set the obtaining condition for the scan data.

The controlling unit may be configured to cause an ultrasound image and the indicator to be displayed side by side, the ultrasound image being based on the scan data of the subject obtained by the obtaining unit.

The controlling unit may be configured to cause the indicator to be displayed, before an ultrasound image based on the scan data of the subject obtained by the obtaining unit is displayed, in response to the obtaining condition for the scan data having been set by the setting unit.

The setting unit may be configured to set the obtaining condition for the scan data, on the basis of at least one of B-mode data and Doppler data of the subject and the analysis result of the shear wave.

On the basis of the analysis result of the shear wave, the setting unit may be configured to set the obtaining condition for the scan data related to at least one of B-mode data and Doppler data of the subject.

The analyzing unit may be configured to analyze a shear wave that has propagated in each of a plurality of mutually-different regions of the subject, and the obtaining condition for the scan data may be set on the basis of the analysis result of the shear wave corresponding to each of the plurality of mutually-different regions.

A method provided in an aspect of the present embodiment includes analyzing a shear wave that has propagated in a subject, setting an obtaining condition for scan data on the basis of an analysis result of the shear wave, performing a scan on the subject on the basis of the obtaining condition, and obtaining the scan data of the subject.

A storage medium provided in an aspect of the present embodiment storing therein, in a non-transitory manner, a program that causes a computer to execute processes of: analyzing a shear wave that has propagated in a subject, setting an obtaining condition for scan data on the basis of an analysis result of the shear wave, and performing a scan on the subject on the basis of the obtaining condition and to obtain the scan data of the subject.

BRIEF DESCRIPTION OF THE DRAWINGS

FIG. 1 is a block diagram illustrating an exemplary configuration of an ultrasound diagnosis apparatus according to a first embodiment;

FIG. 2A is a drawing for explaining a propagation of a shear wave in SWE;

FIG. 2B is a drawing for explaining another propagation of a shear wave in SWE;

FIG. 3 is a flowchart illustrating a procedure in processes performed by the ultrasound diagnosis apparatus according to the first embodiment;

FIG. 4A is a drawing for explaining an example of a process performed by an analyzing function according to the first embodiment;

FIG. 4B is a drawing for explaining another example of the process performed by the analyzing function according to the first embodiment;

FIG. 5A is a drawing for explaining yet another example of the process performed by the analyzing function according to the first embodiment;

FIG. 5B is a drawing for explaining yet another example of the process performed by the analyzing function according to the first embodiment;

FIG. 5C is a drawing for explaining yet another example of the process performed by the analyzing function according to the first embodiment;

FIG. 6 is a drawing for explaining an example of a process performed by a setting function according to the first embodiment;

FIG. 7 is a drawing for explaining a sequence in a process performed by the ultrasound diagnosis apparatus according to the first embodiment;

FIG. 8 is a drawing for explaining processes performed by the ultrasound diagnosis apparatus according to the first embodiment;

FIG. 9A is a drawing for explaining an example in which a shear wave is successfully measured within a region suitable for a measured subject;

FIG. 9B is a drawing for explaining an example in which a shear wave is successfully measured within a time period suitable for a measured subject;

FIG. 10 is a drawing for explaining an indicator indicating a tissue property of an examined subject; and

FIG. 11 is a drawing for explaining a display example of the indicator illustrated in FIG. 10.

DETAILED DESCRIPTION

Exemplary embodiments of an ultrasound diagnosis apparatus, a method, and a program of the present disclosure will be explained in detail below, with reference to the accompanying drawings. It should be noted that the ultrasound diagnosis apparatus, the method, and the program of the present disclosure are not limited by the embodiments described below.

First Embodiment

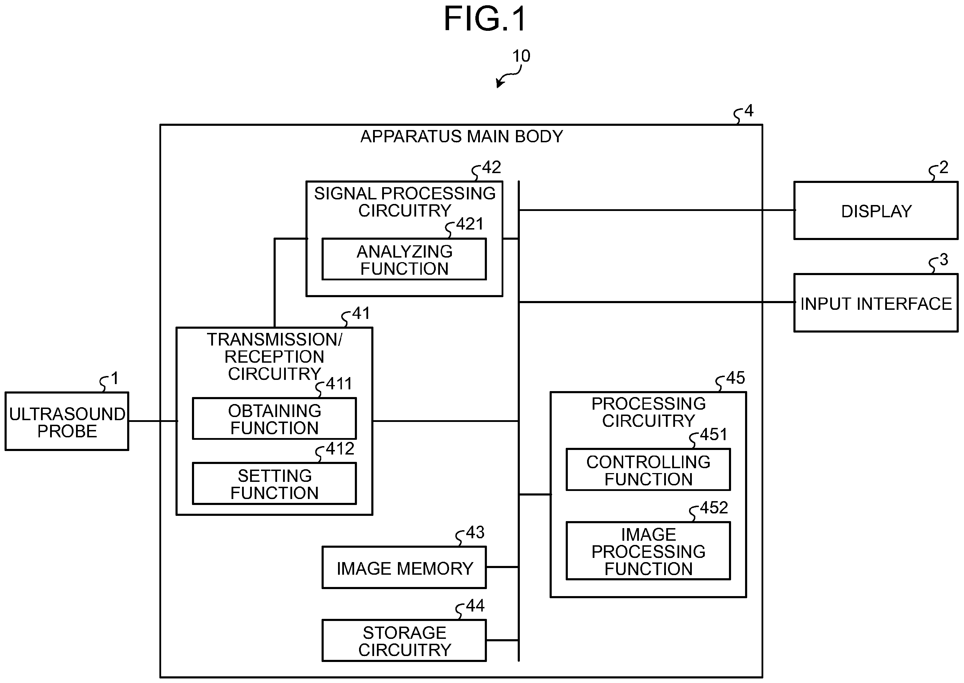

FIG. 1 is a block diagram illustrating an exemplary configuration of an ultrasound diagnosis apparatus 10 according to a first embodiment. As illustrated in FIG. 1, the ultrasound diagnosis apparatus 10 according to the present embodiment includes an ultrasound probe 1, a display 2, an input interface 3, and an apparatus main body 4. The ultrasound probe 1, the display 2, and the input interface 3 are communicably connected to the apparatus main body 4.

The ultrasound probe 1 includes a plurality of piezoelectric transducer elements. The plurality of piezoelectric transducer elements are configured to generate an ultrasound wave on the basis of a drive signal supplied from transmission/reception circuitry 41. Further, the ultrasound probe 1 is configured to receive a reflected wave from an examined subject (hereinafter, "subject") and to convert the reflected wave into an electrical signal. In addition, the ultrasound probe 1 includes a matching layer provided for the piezoelectric transducer elements, a backing member configured to prevent the ultrasound wave from propagating rearwards from the piezoelectric transducer elements, and the like. In this situation, the ultrasound probe 1 is detachably connected to the apparatus main body 4.

When the ultrasound wave is transmitted from the ultrasound probe 1 to the subject, the transmitted ultrasound wave is repeatedly reflected on a surface of discontinuity of acoustic impedances at a tissue in the body of the subject and is received as reflected-wave signals by the plurality of piezoelectric transducer elements included in the ultrasound probe 1. The amplitudes of the received reflected-wave signals are dependent on the difference between the acoustic impedances on the surface of discontinuity on which the ultrasound wave is reflected. Further, when a transmitted ultrasound pulse is reflected on the surface of a moving blood flow, a cardiac wall, or the like, the reflected-wave signals are, due to the Doppler effect, subject to a frequency shift, depending on a velocity component of the moving members with respect to the ultrasound wave transmission direction.

The ultrasound probe 1 may be a one-dimensional ultrasound probe in which the plurality of piezoelectric transducer elements are arranged in a single line. Alternatively, the ultrasound probe 1 may be an ultrasound probe configured to mechanically swing the plurality of piezoelectric transducer elements in a one-dimensional ultrasound probe or may be a two-dimensional ultrasound probe in which the plurality of piezoelectric transducer elements are arranged two-dimensionally in a matrix formation.

The display 2 is configured to display a Graphical User Interface (GUI) used by an operator of the ultrasound diagnosis apparatus 10 for inputting various types of setting requests with the use of the input interface 3 and to display ultrasound images and the like generated by the apparatus main body 4. Further, the display 2 is configured to display various types of messages and display information, to notify the operator of processing statuses and processing results of the apparatus main body 4. Further, the display 2 has a speaker and is also capable of outputting audio.

The input interface 3 is operated for setting a prescribed position (e.g., a position of a Region Of Interest (ROI)) and the like and may be realized, for example, by using a trackball, a switch button, a mouse, a keyboard, a touchpad on which an input operation can be performed by touching an operation surface thereof, a touch monitor in which a display screen and a touchpad are integrally formed, contactless input circuitry using an optical sensor, audio input circuitry, and/or the like. The input interface 3 is connected to processing circuitry 45 (explained later) and is configured to convert an input operation received from the operator into an electrical signal and to output the electrical signal to the processing circuitry 45. In the present disclosure, the input interface 3 does not necessarily need to include physical operation component parts such as the mouse, the keyboard, and/or the like. For instance, possible examples of the input interface include electrical signal processing circuitry configured to receive an electrical signal corresponding to an input operation from an external input machine provided separately from the apparatus and to output the electrical signal to the processing circuitry 45.

The apparatus main body 4 is an apparatus configured to generate the ultrasound image on the basis of the reflected-wave signals received by the ultrasound probe 1 and includes, as illustrated in FIG. 1, the transmission/reception circuitry 41, signal processing circuitry 42, an image memory 43, storage circuitry 44, and the processing circuitry 45. The transmission/reception circuitry 41, the signal processing circuitry 42, the image memory 43, the storage circuitry 44, and the processing circuitry 45 are connected so as to be able to notify one another. In the ultrasound diagnosis apparatus 1 illustrated in FIG. 1, processing functions are stored in the storage circuitry 44 in the form of computer-executable programs. The transmission/reception circuitry 41, the signal processing circuitry 42, and the processing circuitry 45 are processors configured to realize the functions corresponding to the programs, by reading and executing the programs from the storage circuitry 44. In other words, the pieces of circuitry that have read the programs have the functions corresponding to the read programs.

The transmission/reception circuitry 41 includes a pulse generator, a transmission delay unit, a pulser, and the like and is configured to supply the drive signal to the ultrasound probe 1. The pulse generator is configured to repeatedly generate a rate pulse for forming a transmission ultrasound wave at a prescribed rate frequency. The transmission delay unit is configured to apply, to the rate pulses generated by the pulse generator, delay periods that respectively correspond to the piezoelectric transducer elements and are necessary for converging the ultrasound wave generated by the ultrasound probe 1 into the form of a beam and determining transmission directionality. The pulser is configured to apply the drive signal (a drive pulse) to the ultrasound probe 1, with timing based on the rate pulses. In other words, the transmission delay unit is configured to arbitrarily adjust transmission directions of the ultrasound wave transmitted from surfaces of the piezoelectric transducer elements, by varying the delay periods applied to the rate pulses.

Further, to execute a predetermined scan sequence on the basis of an instruction from the processing circuitry 45 (explained later), the transmission/reception circuitry 41 has a function capable of instantaneously changing a transmission frequency, transmission drive voltage, and the like. In particular, the capability to change the transmission drive voltage is realized by transmission circuitry of a linear amplifier type capable of instantaneously switching the value of the transmission drive voltage or a mechanism configured to electrically switch between a plurality of power source units.

Further, the transmission/reception circuitry 41 includes a pre-amplifier, an analog/digital (A/D) converter, a reception delay unit, an adder, and the like and is configured to generate reflected-wave data by performing various types of processes on the reflected-wave signals received by the ultrasound probe 1. The pre-amplifier is configured to amplify the reflected-wave signals with respect to each channel. The A/D converter is configured to perform an A/D conversion on the amplified reflected-wave signals. The reception delay unit is configured to apply a delay period necessary for determining reception directionality. The adder is configured to generate the reflected-wave data by performing an adding process on the reflected-wave signals processed by the reception delay unit. As a result of the adding process by the adder, reflected components from a direction corresponding to the reception directionality of the reflected-wave signals are emphasized, so that a comprehensive beam for the ultrasound transmission/reception is formed on the basis of the reception directionality and the transmission directionality.

In this situation, as illustrated in FIG. 1, the transmission/reception circuitry 41 is configured to execute an obtaining function 411 and a setting function 412. The obtaining function 411 is configured to obtain scan data by transmitting and receiving an ultrasound wave for observing a shear wave of the subject. Further, before the scan data is obtained, the obtaining function 411 is configured to obtain a biological signal of the subject. The setting function 412 is configured to set an obtaining condition for the scan data on the basis of an index. Processes performed by the obtaining function 411 and the setting function 412 will be explained in detail later. The obtaining function 411 is an example of the first obtaining unit and the second obtaining unit. The setting function 412 is an example of the setting unit.

For example, the signal processing circuitry 42 is configured to generate data (B-mode data) in which a signal intensity at each of sample points is expressed with a brightness level, by performing, on the reflected-wave data received from the transmission/reception circuitry 41, a logarithmic amplification, an envelope detection process, and the like. The B-mode data generated by the signal processing circuitry 42 is output to the processing circuitry 45.

Further, for example, the signal processing circuitry 42 is configured to generate data (Doppler data) obtained by extracting movement information based on the Doppler effect of the moving members, from the reflected-wave data received from the transmission/reception circuitry 41, at each of the sample points in a scan region. More specifically, the signal processing circuitry 42 is configured to perform a frequency analysis to obtain velocity information from the reflected-wave data, to extract a blood flow, a tissue, and a contrast agent echo component under the Doppler effect, and to generate the data (the Doppler data) obtained by extracting moving member information such as an average velocity, a dispersion value, a power value, and the like with respect to multiple points. In this situation, the term moving members refers to the blood flow, the tissue such as a cardiac wall, and the contrast agent, for example. The movement information (blood flow information) obtained by the signal processing circuitry 42 is forwarded to the processing circuitry 45 and is displayed in color on the display 2 as an average velocity image, a dispersion image, a power image, or an image combining any of these images together.

Further, as illustrated in FIG. 1, the signal processing circuitry 42 is configured execute an analyzing function 421. The analyzing function 421 is configured to calculate the index for determining the obtaining condition for the scan data, on the basis of the biological signal. Processes performed by the analyzing function 421 will be explained in detail later. The analyzing function 421 is an example of the analyzing unit.

The image memory 43 is a memory configured to store therein display-purpose image data generated by the processing circuitry 45. Further, the image memory 43 is also capable of storing therein any of the data generated by the signal processing circuitry 42. The operator is able to invoke the B-mode data and the Doppler data stored in the image memory 43 after a diagnosis process, for example. The invoked data serves as a display-purpose ultrasound image after being routed through the processing circuitry 45.

The storage circuitry 44 is configured to store therein control programs for performing the ultrasound transmission/reception, image processing processes, and display processes, as well as various types of data such as diagnosis information (e.g., patient IDs, medical doctors' observations, etc.), diagnosis protocols, and various types of data such as various types of body marks. Further, the storage circuitry 44 is also configured to store therein processing results of the transmission/reception circuitry 41, the signal processing circuitry 42, and the processing circuitry 45. In addition, the storage circuitry 44 may also be used for saving any of the image data stored in the image memory 43 and the like, as necessary. Further, any of the data stored in the storage circuitry 44 may be transferred to an external apparatus via an interface (not illustrated). The storage circuitry 44 is an example of the storage unit.

The processing circuitry 45 is configured to control entire processes performed by the ultrasound diagnosis apparatus 10. More specifically, on the basis of the various types of setting requests input by the operator via the input interface 3 and the various types of control programs and the various types of data read from the storage circuitry 44, the processing circuitry 45 is configured to control processes performed by the transmission/reception circuitry 41 and the signal processing circuitry 42. Further, the processing circuitry 45 is configured to control the display 2 so as to display the display-purpose ultrasound image stored in the image memory 43.

As illustrated in FIG. 1, the processing circuitry 45 is configured to execute a controlling function 451 and an image processing function 452. In this situation, the controlling function 451 is an example of a display controlling unit.

The controlling function 451 is configured to control the processes performed by the transmission/reception circuitry 41 and the signal processing circuitry 42, on the basis of the various types of setting requests input by the operator via the input interface 3 and the various types of control programs and the various types of data read from the storage circuitry 44. Further, the controlling function 451 is configured to control the display 2 so as to display ultrasound images and various types of information. For example, the controlling function 451 is configured to exercise control so that a display unit displays the index.

The image processing function 452 is configured to generate ultrasound images from the data generated by the signal processing circuitry 42. In other words, the image processing function 452 is configured to generate an ultrasound image in which the intensities of the reflected waves are expressed with the brightness levels, from the B-mode data generated by the signal processing circuitry 42. Further, the image processing function 452 is configured to generate an ultrasound image indicating the moving member information (the blood flow information and moving information of the tissue) from the Doppler data generated by the signal processing circuitry 42. The ultrasound image based on the Doppler data may be velocity image data, dispersion image data, power image data, or image data combining any of these image data together.

In this situation, generally speaking, the image processing function 452 is configured to convert (by performing a scan convert process) a scanning line signal sequence from an ultrasound scan into a scanning line signal sequence in a video format used by television, for example, and to generate the display-purpose ultrasound image. More specifically, the image processing function 452 is configured to generate the display-purpose ultrasound image by performing a coordinate transformation process compliant with an ultrasound scanning mode used by the ultrasound probe 1. Further, as various types of image processing processes besides the scan convert process, the image processing function 452 is configured to perform, for example, an image processing process (a smoothing process) to re-generate an average brightness value image, an image processing process (an edge enhancement process) that uses a differential filter inside an image, or the like, by using a plurality of image frames resulting from the scan convert process. Also, the image processing function 452 is configured to combine text information of various types of parameters, scale graduations, body marks, and the like with the ultrasound image.

In other words, the B-mode data and the Doppler data are ultrasound image data before the scan convert process, whereas the data generated by the image processing function 452 is the display-purpose ultrasound image data resulting from the scan convert process. In this situation, when the signal processing circuitry 42 has generated three-dimensional data (three-dimensional B-mode data and three-dimensional Doppler data), the image processing function 452 is configured to generate volume data by performing a coordinate transformation process in accordance with the ultrasound scanning mode used by the ultrasound probe 1. Further, by performing any of various types of rendering processes on the volume data, the image processing function 452 is configured to generate display-purpose two-dimensional image data.

In this situation, the ultrasound diagnosis apparatus 10 according to the first embodiment is an apparatus capable of carrying out elastography by which firmness (an elastic modulus or the like) of a biological tissue is measured and a distribution of measured firmness levels is visualized. More specifically, the ultrasound diagnosis apparatus 10 according to the first embodiment is an apparatus capable of carrying out Shear Wave Elastography (SWE) by causing a displacement in the biological tissue by applying acoustic radiation force thereto.

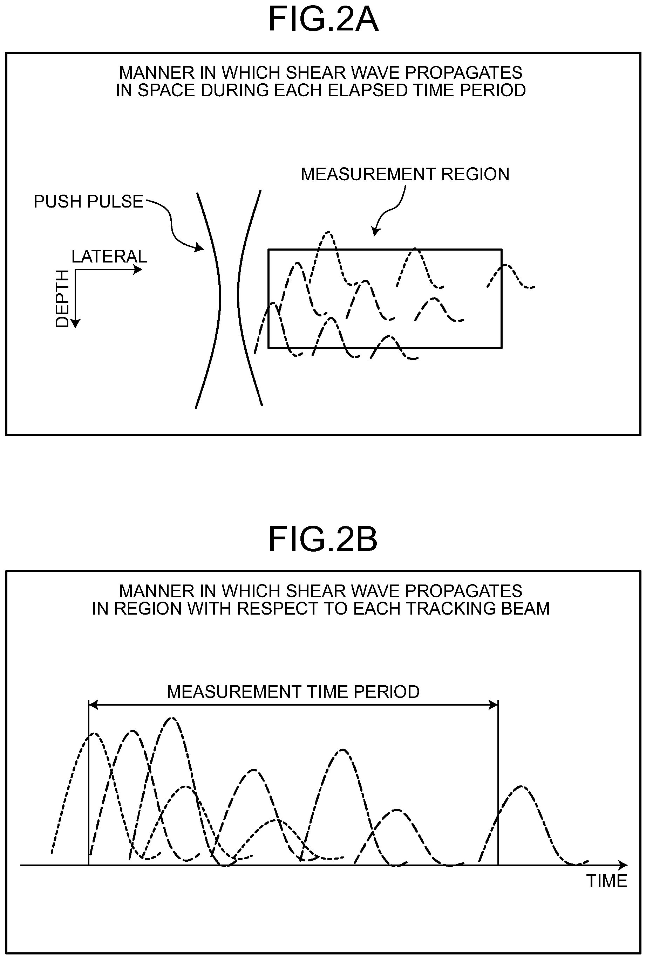

In a current state of technology, SWE is configured to measure firmness of a tissue serving as a measured subject, by using a single condition. Thus, in some situations, it may not be possible to properly observe a shear wave, depending on the state of the measured subject. FIGS. 2A and 2B are drawings for explaining propagations of shear waves in SWE. FIG. 2A is a drawing for explaining a propagation of a shear wave in a space where a push pulse is transmitted, during each elapsed time period. FIG. 2B is a drawing for explaining a propagation of a shear wave in a measurement space where the firmness is measured, with respect to each tracking pulse.

As illustrated in FIG. 2A, when the push pulse is transmitted to the biological tissue, a shear wave propagating in a lateral direction occurs. In this situation, according to SWE in the current state of technology, because the shear wave is observed under a single obtaining condition, there may be some situations where the shear wave may not be contained within the measurement region, depending on the firmness of the tissue in the measurement region. In other words, when the tissue serving as the measured subject is firm, because the shear wave has a high propagation velocity as indicated with the dotted-line waveform in the drawing, there may be some situations where it is not possible to properly grasp the shear wave with tracking pulses. On the contrary, when the tissue serving as the measured subject is soft, because the shear wave has a low propagation velocity as indicated with the dashed-chain-line waveform in the drawing, there may be some situations where it is not possible to properly grasp the shear wave with tracking pulses. In contrast, when a shear wave has a waveform such as that indicated with the broken line, it is possible to properly observe the shear wave.

Further, as illustrated in FIG. 2B, according to SWE in the current state of technology, there may be some situations where, in a measurement region, a shear wave may not be contained within a measurement period from a measurement start to a measurement end. In other words, when the tissue serving as a measured subject is firm, because the shear wave has a high propagation velocity, there may be some situations where the timing to start the measuring process may be too late as indicated with the dotted-line waveform in the drawing, and it may not be possible to properly grasp the shear wave. On the contrary, when the tissue serving as a measured subject is soft, because the shear wave has a low propagation velocity, there may be some situations where the propagation of the shear wave may be too late for the timing to end the measuring process as indicated with the dashed-chain-line waveform in the drawing, and it may not be possible to properly grasp the shear wave. In contrast, when a shear wave has a waveform such as that indicated with the broken line, it is possible to properly observe the shear wave.

As explained above, in the SWE measuring processes, as for the push pulse for generating the shear wave and the tracking pulses for observing the shear wave, optimal transmission/reception conditions may vary depending on the firmness of the tissue being measured. To cope with this situation, the ultrasound diagnosis apparatus 10 according to the first embodiment is configured to calculate approximate information related to the firmness by performing a pre-scan on the measured subject and to further set an obtaining condition on the basis of the calculated information and thus makes it possible to perform the measuring process by using the condition suitable for the measured subject.

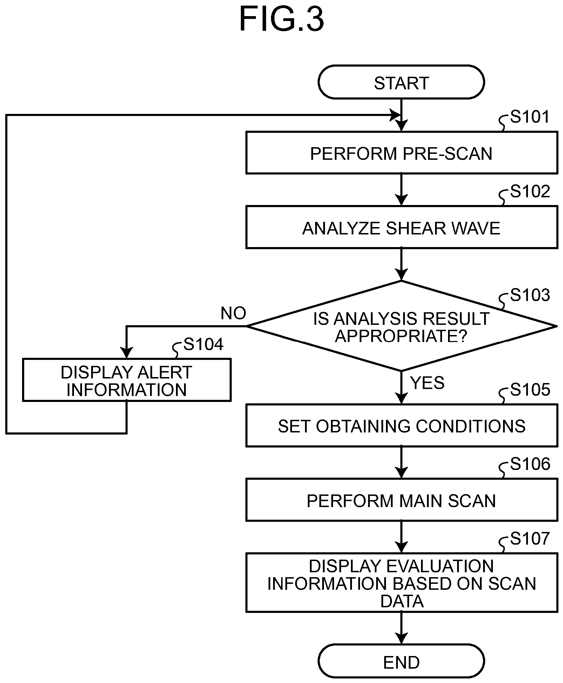

Next, a procedure in processes performed by the ultrasound diagnosis apparatus 10 will be explained with reference to FIG. 3, before details of the processes are explained. FIG. 3 is a flowchart illustrating the procedure in the processes performed by the ultrasound diagnosis apparatus according to the first embodiment.

For example, as illustrated in FIG. 3, in the present embodiment, the obtaining function 411 performs a pre-scan (pre SWE) on the subject (step S101). More specifically, the obtaining function 411 performs transmission of a push pulse and transmission/reception of tracking pulses and thereby obtains scan data (a biological signal of the subject) related to a shear wave that has propagated in the subject. The process at step S101 described above is realized, for example, as a result of the transmission/reception circuitry 41 invoking and executing the program corresponding to the obtaining function 411 from the storage circuitry 44.

Subsequently, the analyzing function 421 analyzes the shear wave that has propagated in the subject, on the basis of the scan data obtained by the obtaining function 411 (step S102). The analyzing function 421 analyzes the shear wave (a first shear wave) that has propagated through various positions in a measurement region (a first measurement region R1) of the subject targeted for the pre SWE. The analyzing function 421 obtains tissue property index values indicating a tissue property of the subject in each of the various positions in the first measurement region R1, as an analysis result of the shear wave. After that, the analyzing function 421 judges whether or not the analysis result is appropriate (step S103). The processes at steps S102 and S103 described above are realized, for example, as a result of the signal processing circuitry 42 invoking and executing the program corresponding to the analyzing function 421 from the storage circuitry 44.

In the judgment at step S103, when the analysis result is not appropriate (step S103: No), the controlling function 451 causes the display 2 to display alert information (step S104) and exercises control so that a pre-scan is performed again. The process at step S104 described above is realized, for example, as a result of the processing circuitry 45 invoking and executing the program corresponding to the controlling function 451 from the storage circuitry 44.

On the contrary, in the judgment at step S103, when the analysis result is appropriate (step S103: Yes), the setting function 412 sets obtaining conditions for scan data in SWE, on the basis of the analysis result of the shear wave (step S105). More specifically, on the basis of the analysis result of the first shear wave, the setting function 412 sets, as the obtaining conditions for the scan data, a push pulse transmission condition for causing a second shear wave to occur in the subject and a tracking pulse transmission/reception condition for observing the second shear wave. The setting function 412 sets the obtaining conditions for the scan data on the basis of the tissue property index values in the various positions within the first measurement region R1. The process at step S105 described above is realized, for example, as a result of the transmission/reception circuitry 41 invoking and executing the program corresponding to the setting function 412 from the storage circuitry 44.

Subsequently, the obtaining function 411 performs a main scan (a main SWE) by using the obtaining conditions that were set (step S106). In other words, the obtaining function 411 obtains the scan data of the subject by performing the main scan on the subject, on the basis of the obtaining conditions set by the setting function 412. The process at step S106 described above is realized, for example, as a result of the transmission/reception circuitry 41 invoking and executing the program corresponding to the obtaining function 411 from the storage circuitry 44.

Subsequently, the controlling function 451 exercises control so as to cause the display 2 to display evaluation information (the firmness of the tissue serving as the measured subject) based on the scan data (step S107). In other words, the controlling function 451 displays, as the evaluation information, an ultrasound image (a SWE image) based on the scan data of the subject obtained by the obtaining function 411. The process at step S106 described above is realized, for example, as a result of the processing circuitry 45 invoking and executing the program corresponding to the controlling function 451 from the storage circuitry 44.

Next, details of the processes performed by the ultrasound diagnosis apparatus 10 will be explained.

The pre-scan

As explained at step S101, the obtaining function 411 is configured to perform the pre-scan (the pre SWE) including the transmission of the push pulse and the transmission/reception of the tracking pulses. In this situation, the push pulse is a convergent ultrasound pulse that causes a transversal wave referred to as the shear wave in a biological tissue (the subject) on the basis of the acoustic radiation force and is an example of an ultrasound wave for causing the shear wave. Further, the tracking pulses are ultrasound pulses for observing the shear wave and serve as an example of an ultrasound wave for observing the shear wave.

For example, the obtaining function 411 is configured to cause the push pulse to be transmitted from the ultrasound probe 1 so that the first shear wave occurs in the biological tissue. After that, the obtaining function 411 is configured to cause the ultrasound probe 1 to transmit the tracking pulses for observing the first shear wave that occurred on the basis of the push pulse. The tracking pulses are transmitted for observing propagation velocities of the first shear wave generated by the push pulse, at the sample points within the first measurement region R1. Normally, a tracking pulse is transmitted multiple times (e.g., 100 times) with respect to the scanning lines within the first measurement region R1. The obtaining function 411 is configured to generate the reflected-wave data (the scan data) from reflected-wave signals of the tracking pulses transmitted with respect to the scanning lines within the first measurement region R1.

In this situation, the obtaining function 411 is configured, in the pre-scan, to carry out the push pulse transmission and the tracking pulse transmission/reception, by using the obtaining conditions under which it is possible to obtain the shear waves propagating in tissues having mutually-different tissue properties. In other words, the obtaining function 411 is configured to carry out the pre-scan by using the push pulse transmission condition and the tracking pulse transmission/reception condition with which it is possible to grasp the shear wave in the presence of the various tissue properties (a tissue having various levels of firmness). The obtaining conditions for the pre-scan (the obtaining conditions for the scan data) are set in advance and stored in the storage circuitry 44 so as to be read by the obtaining function 411 at the time of the pre-scan.

The analyzing process

As explained at step S102, the analyzing function 421 is configured to calculate a level of firmness (the elastic modulus) of the tissue in the first measurement region R1, by analyzing the shear wave (the first shear wave) obtained in the pre-scan (the pre SWE) performed by the obtaining function 411. More specifically, the analyzing function 421 is configured to analyze the shear wave (the first shear wave) that has propagated through the various positions within the measurement region (the first measurement region R1) of the subject targeted for the pre SWE. The analyzing function 421 is configured to obtain, as the analysis result of the first shear wave, the tissue property index values indicating the tissue properties of the subject in the various positions within the first measurement region R1.

For example, the tissue property index values may be at least one selected from between: elasticity index values indicating elasticity of the tissue of the subject; and viscosity index values indicating viscosity of the tissue of the subject. Examples of the elasticity index values (elasticity values) include a propagation velocity of the shear wave, an arrival time of the shear wave, a shear elastic modulus, and Young's modulus. Examples of the viscosity index values (viscosity values) include viscosity coefficients calculated by using a Voigt model or a Maxwell model on a relationship between frequency components and the propagation velocities (velocities) and dispersion values (dispersion slope values) of the propagation velocities with respect to the frequency components. Further, the analyzing function 421 may be configured to calculate a statistical value (e.g., an average value, a median, a variance, a standard deviation value, or a range of the tissue property index values) of the tissue property index values in the various positions within the first measurement region R1, as an approximate tissue property index value in the first measurement region R1 and to further use the calculated value as the analysis result of the first shear wave.

For example, the analyzing function 421 is configured to calculate firmness distribution data (the elasticity index values in the various positions within the first measurement region R1) indicating a distribution of firmness in the first measurement region R1, by analyzing the reflected-wave data of the tracking pulses transmitted multiple times with respect to the scanning lines within the first measurement region R1 in the pre-scan. In an example, the analyzing function 421 may be configured to measure the propagation velocity of the first shear wave generated by the push pulse at each of the sample points within the first measurement region R1 and to calculate a statistical value (an average value, a median, a variance, a standard deviation value, or a range of propagation velocities) of the propagation velocities (the elasticity index values) at the sample points, as the analysis result of the first shear wave.

For example, the analyzing function 421 is configured to generate movement information (tissue Doppler data) over a plurality of temporal phases, with respect to each of the plurality of sample points on the scanning lines, by performing a frequency analysis on the reflected-wave data of the tracking pulses. After that, the analyzing function 421 is configured to perform time integration on velocity components of the tissue Doppler data over the plurality of temporal phases obtained at each of the plurality of sample points on the scanning lines. In this manner, the analyzing function 421 is configured to calculate a displacement over the plurality of temporal phases at each of the plurality of sample points on the scanning lines. In other words, the displacements are detected as a displacement waveform (a time displacement curve). That is to say, the analyzing function 421 is configured to detect movements (the displacements) of the tissue in each of the plurality of positions in the subject, by analyzing the scan data acquired through the push pulse transmission and the tracking pulse transmission/reception. Further, the displacement waveform is an example of waveform information expressing the shear wave.

Subsequently, the analyzing function 421 is configured to obtain a time at which the displacement is at a maximum at each of the sample points in the first measurement region R1. Further, the analyzing function 421 is configured to determine the time at which the maximum displacement was acquired at each of the sample points, as an arrival time at which the shear wave arrived at the sample point. Subsequently, the analyzing function 421 is configured to calculate the propagation velocities of the first shear wave at the sample points, by performing a spatial differential on the shear wave arrival times at the sample points. In this situation, the first shear wave arrival times are not limited only to the time at which the displacement is at a maximum at each of the sample points. Alternatively, for example, it is acceptable to use a time at which a change amount in the displacement is at a maximum at each of the sample points.

Further, the analyzing function 421 is configured to calculate an approximate elasticity value (e.g., a statistical value of the elasticity index values) in the first measurement region R1 from the information about the propagation velocities of the first shear wave at the sample points in the first measurement region R1. Shear waves have a high propagation velocity in a firm tissue and have a low propagation velocity in a soft tissue. In other words, the values of the propagation velocities of the shear wave serve as values (elasticity index values) indicating firmness of the tissue. In the above example, the tracking pulses are transmission pulses for tissue Doppler. Alternatively, the propagation velocities of the shear wave may be calculated from a mutual correlation in tissue displacements between scanning lines positioned adjacent to each other.

Further, the analyzing function 421 may calculate an elastic modulus (e.g., Young's modulus, a shear elastic modulus) from the propagation velocities of the first shear wave. The propagation velocities, Young's modulus, and the shear elastic modulus of the shear wave may all be used as a physical quantity (an elasticity index value) expressing firmness of the biological tissue. The firmness is an example of a parameter expressing a tissue property (the elasticity).

Alternatively, instead of calculating the approximate elasticity value (e.g., a statistical value of the elasticity index values) in the first measurement region R1 on the basis of the propagation velocities of the first shear wave, the analyzing function 421 may calculate an approximate elasticity value in the first measurement region R1 on the basis of waveform information of the first shear wave. A calculation example in each situation will be explained below.

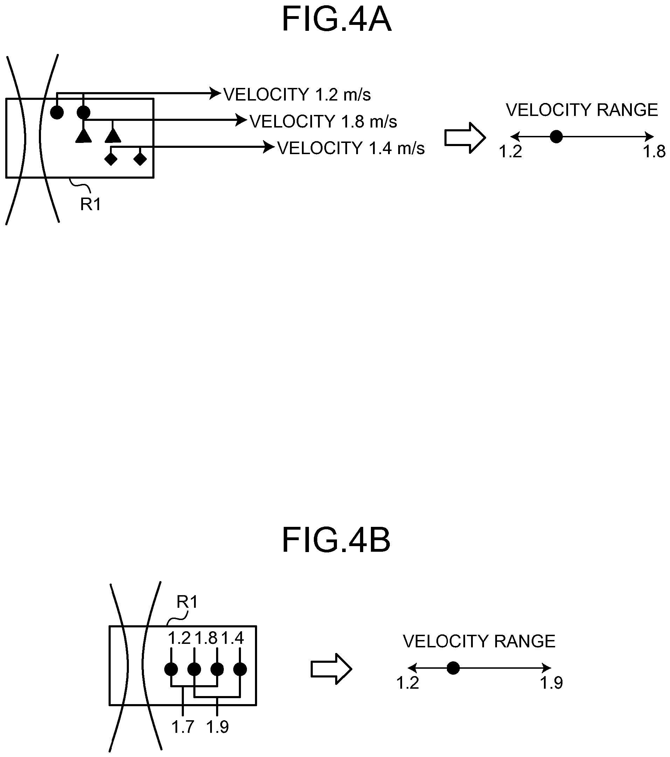

At first, an example in which the approximate elasticity value in the first measurement region R1 is calculated on the basis of the propagation velocities of the first shear wave will be explained. FIGS. 4A and 4B are drawings for explaining examples of processes performed by the analyzing function 421 according to the first embodiment. In this situation, FIGS. 4A and 4B illustrate examples in which a statistical value of the elasticity index values in the first measurement region R1 is calculated, on the basis of the propagation velocities of the first shear wave obtained at various positions in the first measurement region R1 by performing the pre-scan.

For example, as illustrated in FIG. 4A, by analyzing the reflected-wave data of the tracking pulses transmitted multiple times with respect to the scanning lines in the first measurement region R1 (the square in the drawing) during the pre-scan, the analyzing function 421 is configured to calculate a propagation velocity at each of the positions in the first measurement region R1, with regard to the first shear wave generated by the push pulse. In an example, as illustrated in FIG. 4A, the analyzing function 421 calculates propagation velocities "1.2 m/s", "1.8 m/s", and "1.4 m/s" between adjacently-positioned scanning lines. After that, from the calculated propagation velocities, the analyzing function 421 is configured to calculate a range of propagation velocities of the first shear wave in the first measurement region R1 as a "velocity range: 1.2 m/s to 1.8 m/s". In other words, in this situation, the "velocity range: 1.2 m/s to 1.8 m/s" is obtained as the approximate elasticity value (a statistical value of the propagation velocities) in the first measurement region R1 illustrated in FIG. 4A.

Further, the analyzing function 421 is capable of analyzing the propagation velocities of the first shear wave, not only for the spaces between the adjacently-positioned scanning lines but also for other positions. For example, as illustrated in FIG. 4B, in addition to the propagation velocities "1.2 m/s", "1.8 m/s", and "1.4 m/s" of the first shear wave between the adjacently-positioned scanning lines, the analyzing function 421 may also calculate propagation velocities "1.7 m/s" and "1.9 m/s" of the first shear wave between scanning lines that are not positioned adjacent to each other and may further calculate a "velocity range: 1.2 m/s to 1.9 m/s" in the first measurement region R1 from the calculated propagation velocities. In other words, in this situation, the "velocity range: 1.2 m/s to 1.9 m/s" is obtained as an approximate elasticity value (a statistical value of the tissue property index values) in the first measurement region R1 illustrated in FIG. 4B.

Further, the above examples are merely examples, and possible embodiments are not limited to those examples. For instance, the quantity of the propagation velocities to be calculated by the analyzing function 421 is not limited to that illustrated in the drawings. The above sections describe the situation in which the range of the elasticity values in the first measurement region R1 is calculated as an example of the approximate elasticity value (the statistical value of the elasticity index values) in the first measurement region R1; however, the analyzing function 421 may be configured to calculate a statistical value such as an average value, a median, a variance, a standard deviation value, or the like of the elasticity values, as an approximate elasticity value in the first measurement region R1. For example, the analyzing function 421 may be configured to calculate an average value or a median of the propagation velocities of the first shear wave in the various positions in the first measurement region R1 as illustrated in FIG. 4A or FIG. 4B and to obtain the average value or the median as the approximate elasticity value in the first measurement region R1.

Further, the analyzing function 421 may be configured to adjust the average value, the median, or the range of the elasticity values, by calculating a variance of the elasticity values. For example, the analyzing function 421 may be configured to calculate the variance of the propagation velocities of the first shear wave and, if the calculated variance exceeds a threshold value, to adjust the average value, the median, or the range of the propagation velocities of the first shear wave, so as to obtain an elasticity value corresponding to the adjusted velocity. In an example, when the calculated variance exceeds the threshold value, the analyzing function 421 may be configured to increase the average value or the median or to increase an upper limit value of the range of the propagation velocities of the first shear wave. In other words, when the variance is large, the analyzing function 421 may be configured to change the conditions so that the tissue in the first measurement region R1 includes a firmer tissue portion.

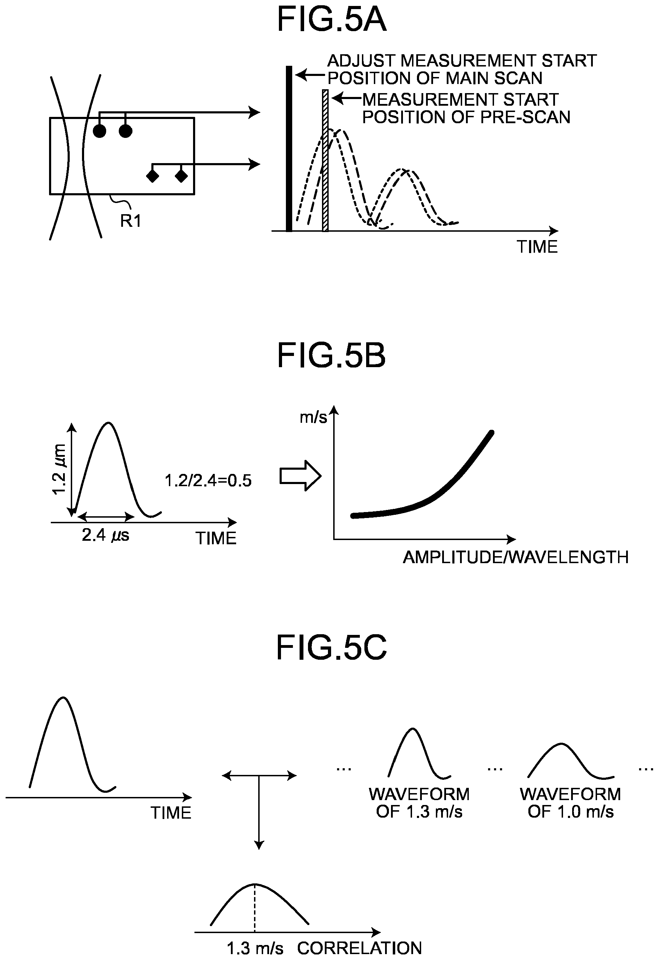

Next, an example will be explained in which the approximate elasticity value in the first measurement region R1 is calculated on the basis of the waveform information of the first shear wave. FIGS. 5A, 5B, and 5C are drawings for explaining examples of processes performed by the analyzing function 421 according to the first embodiment. In this situation, FIGS. 5A, 5B, and 5C illustrate the examples in which a statistical value of the elasticity index values in the first measurement region R1 is calculated on the basis of the waveform information of the shear wave in various positions in the first measurement region R1, by performing a pre-scan.

For example, as illustrated in FIG. 5A, the analyzing function 421 is configured to judge, in the pre-scan, whether or not the arrival times of the first shear wave are contained in the measurement period on the basis of the waveform information of the first shear wave and to calculate a range of elasticity values (a statistical value of the elasticity index values) of the tissue in the first measurement region R1 on the basis of a result of the judgment.

Further, for example, as illustrated in FIG. 5B, the analyzing function 421 may be configured to calculate an elasticity value from an amplitude and a wavelength of the shear wave. In an example, as illustrated in FIG. 5B, when the shear wave has an amplitude of "1.2 $\mu$m" and a wavelength of "2.4 $\mu$s", the analyzing function 421 may calculate a propagation velocity of "1.3 m/s" from "1.2 / 2.4 = 0.5" on the basis of a mathematical function indicating a relationship between propagation velocities "m/s" and "amplitude/wavelength". After that, for example, the analyzing function 421 obtains a firmness value "7 kPa" from the propagation velocity "1.3 m/s". In this situation, by obtaining a firmness value as described above with respect to a plurality of positions in the first measurement region R1, the analyzing function 421 is able to calculate an average value, a median, and a range (statistical values of the elasticity index values) of the elasticity values in the first measurement region R1. As for the wavelengths and the amplitudes of the first shear wave, a softer tissue exhibits a higher amplitude and a longer wavelength, whereas a firmer tissue exhibits a lower amplitude and a shorter wavelength. Thus, the mathematical function indicating the relationship between the propagation velocities "m/s" and "amplitude/wavelength" is obtained in advance and is stored in the storage circuitry 44, for example.

Further, for example, as illustrated in FIG. 5C, the analyzing function 421 may be configured to calculate an elasticity value from a degree of matching between a waveform of the shear wave and waveform patterns of firmness levels incorporated in advance. In an example, as illustrated in FIG. 5C, the analyzing function 421 may calculate each of mutual correlations between waveforms corresponding to a plurality of propagation velocities including "1.3 m/s" and "1.0 m/s" and the waveform of the shear wave obtained in the pre-scan, so as to determine, from the calculated results of the mutual correlations, that the waveform of the shear wave obtained from the pre-scan has a high degree of matching with the waveform of "1.3 m/s". Further, for example, the analyzing function 421 obtains a firmness value "7 kPa" from the propagation velocity "1.3 m/s". In this situation, by obtaining a firmness level as described above with respect to a plurality of positions in the first measurement region R1, the analyzing function 421 is able to calculate an average value, a median, and a range (statistical values of the elasticity index values) of the elasticity values in the first measurement region R1. Further, the waveform patterns corresponding to the propagation velocities are obtained in advance and stored in the storage circuitry 44, for example.

The analysis result judging process

As explained at step S103, the analyzing function 421 is configured to judge whether or not the analysis result of the first shear wave is appropriate. For example, when the level of any of the calculated elasticity values exhibits an apparently abnormal value or when the variance of the elasticity values exceeds a prescribed threshold vale, the analyzing function 421 determines that the analysis result is not appropriate. In this situation, a reference value or a prescribed threshold value used for judging such abnormal values are set in advance. Further, the prescribed threshold value to be compared with the variance is set to be larger than the threshold value used for judging whether or not the elasticity values should be adjusted as described above.

Displaying the alert information

As explained at step S104, when the analysis result of the first shear wave is determined as being not appropriate, the controlling function 451 is configured to exercise control so that the alert information is displayed. In other words, when the index (the analysis result of the first shear wave) does not satisfy a reference level, the controlling function 451 is configured to exercise control so that the alert information related to the index is displayed. For example, the controlling function 451 may control the display 2 so as to display the alert information indicating that the analysis in the pre-scan was not properly performed. Further, in addition to the abovementioned information, the controlling function 451 may also display information suggesting that the position of the first measurement region R1 should be changed.

Further, the controlling function 451 may also cause the display 2 to display the index (the analysis result of the first shear wave). For example, the controlling function 451 may cause the display 2 to display the elasticity value (the statistical value of the elasticity index values in the present example) in the first measurement region R1 calculated by the analyzing function 421. In this situation, the controlling function 451 may cause the display 2 to display the elasticity values in the first measurement region R1, not only when the alert information is displayed, but also when the analysis result is appropriate.

The obtaining condition setting process

As explained at step S105, when the analysis result is appropriate, the setting function 412 is configured to set the obtaining condition for the scan data in the main scan. More specifically, on the basis of the approximate elasticity value (the statistical value of the elasticity index values in the first measurement region R1 in the present example) calculated by the analyzing function 421, the setting function 412 is configured to set the obtaining condition for the main scan. In other words, on the basis of the analysis result of the first shear wave, the setting function 412 is configured to set a push pulse transmission condition for causing the shear wave (the second shear wave) in the subject and a tracking pulse transmission/reception condition for observing the second shear wave that has propagated in the subject, as the obtaining conditions for the scan data. The setting function 412 according to the present embodiment is capable of setting the obtaining conditions by using various methods. In the following sections, examples thereof will be explained.

For example, on the basis of the index (the analysis result of the first shear wave), the setting function 412 may be configured to select the obtaining conditions for the scan data (the obtaining conditions for the main scan) from among a plurality of obtaining conditions set in advance. In other words, by comparing the approximate elasticity value (the analysis result of the first shear wave (the statistical value of the elasticity index values in the present example)) calculated by the analyzing function 421 with the plurality of obtaining conditions set in advance, the setting function 412 may be configured to select the obtaining conditions suitable for the tissue of the subject on which the pre-scan was performed, from among the plurality of obtaining conditions.

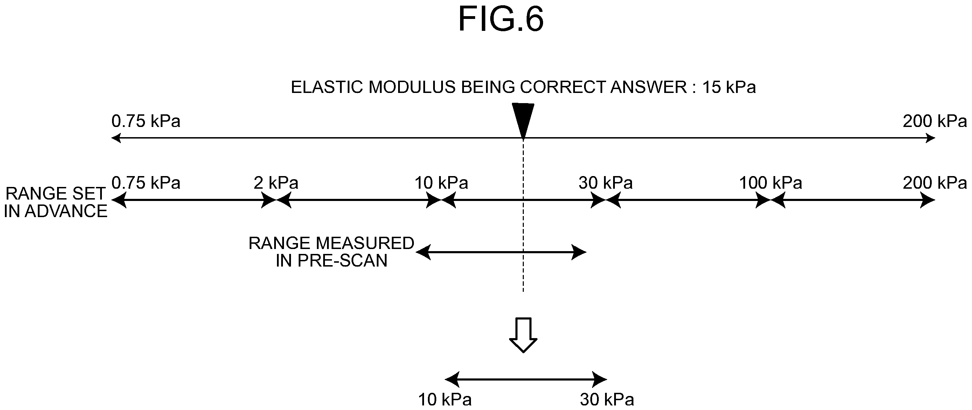

FIG. 6 is a drawing for explaining an example of a process performed by the setting function 412 according to the first embodiment. In the present example, FIG. 6 illustrates a situation in which a range "0.75 kPa to 200 kPa" of elasticity values (firmness) is divided into five ranges set in advance such as "0.75 kPa to 2 kPa", "2 kPa to 10 kPa", "10 kPa to 30 kPa", "30 kPa to 100 kPa", and "100 kPa to 200 kPa", so that an obtaining condition corresponding to each of the divided ranges is set.

For example, as illustrated in FIG. 6, the setting function 412 is configured to select, from among the five ranges set in advance, the range "10 kPa to 30 kPa" corresponding to a statistical value (e.g., an average value, a median, a range, etc.) of the elasticity values in the first measurement region R1 measured in the pre-scan. Alternatively, the setting function 412 may select, from among the five ranges, a range that overlaps the most with the range of the elasticity values in the first measurement region R1 measured in the pre-scan. In this manner, for example, the setting function 412 is able to set obtaining conditions for the range including the elasticity value "15 kPa" being a correct answer.

Although the example was explained with reference to FIG. 6 in which the five ranges are set in advance, possible embodiments are not limited to this example. It is possible to set the ranges in an arbitrary quantity with arbitrary numerical value ranges. Further, although the example was explained with reference to FIG. 6 in which the five ranges set in advance are set so that none of the ranges overlaps with the adjacent range, possible embodiments are not limited to this example. It is also acceptable to set the ranges so that elasticity values overlap between adjacent ranges.

As explained above, the setting function 412 is configured to set the obtaining conditions for the main scan (the obtaining conditions for the scan data), on the basis of the index (the analysis result of the first shear wave) obtained by performing the pre-scan. More specifically, as the obtaining conditions for the scan data in the main scan, the setting function 412 is configured to set at least one selected from among: a transmission condition of the push pulse for observing the second shear wave and a transmission/reception condition of the tracking pulses for observing the second shear wave that has propagated in a second measurement region R2 of the subject targeted for the main SWE; a condition related to a display based on the scan data; and a condition related to an analysis based on the scan data. For example, on the basis of the analysis result of the first shear wave, the setting function 412 may be configured to set a condition including at least one of the position and the size of the second measurement region R2, as the obtaining condition for the scan data. Further, on the basis of the analysis result of the first shear wave, the setting function 412 may be configured to set, as the obtaining condition for the scan data, a condition including at least one selected from among: a measurement start time for measuring the second shear wave in various positions in the second measurement region R2; a measurement end time for the second shear wave; and a measurement time span. For example, the obtaining conditions set by the setting function 412 may include adjustment parameters described below.

Adjustment parameters related to the push pulse include a "transmission velocity", "the number of times of transmission", a "transmission position", a "transmission waveform", and a "framerate". Further, adjustment parameters related to the tracking pulses include a "distance from the push pulse", a "width in the transversal direction (a distance between the scanning lines)", a "measurement start time", a "measurement end time", a "time span", and "the number of simultaneous receptions (the number of reception beams)". Adjustment parameters related to a color bar include at least "changing a color range". Further, adjustment parameters related to the tissue property analysis include at least a "region in which a directional filter is set to 0".

Further, on the basis of the index (the analysis result of the first shear wave), the setting function 412 is configured to set at least one selected from among: the time period from when the biological signal is obtained to when the obtainment of the scan data is started; and the time period until the obtainment of the next scan data is started after the scan data is obtained. In other words, on the basis of the approximate elasticity value in the first measurement region R1 calculated by performing the pre-scan, the setting function 412 is configured to set the time period until the main scan is started after the pre-scan and/or the time period between the scans when a scan is to be performed again after the main scan. In the following sections, the time period until the main scan is started after the pre-scan and the time period until a scan is performed again after the main scan is finished may each be referred to as cool down time.

Next, specific examples of setting the parameters described above will be explained. For example, as the firmness of a tissue becomes softer (as the propagation velocity of the shear wave becomes lower), the various types of adjustment parameters may be set as follows: For example, for the push pulse, the condition may be set so as to "lower the transmission output" as the firmness becomes softer. Further, for example, for the tracking pulses, the condition may be set so as to "increase the distance between the beams (the scanning lines)", to "decrease the distance between the push pulse and the first beam", and/or to "decrease a PRF (make the time spans coarser)", as the firmness becomes softer. Further, the obtaining condition for the scan data may be set so as to "increase a framerate of the push pulse in a multi shot mode" and/or to "shorten the cool down time", as the firmness becomes softer.

In contrast, as the firmness of a tissue becomes firmer (as the propagation velocity of the shear wave becomes higher), the various types of adjustment parameters may be set as follows: For example, for the push pulse, the condition may be set so as to "increase the transmission output" as the firmness becomes firmer. Further, for example, for the tracking pulses, the condition may be set so as to "decrease the distance between the beams (the scanning lines)", to "increase the distance between the push pulse and the first beam", and/or to "increase the PRF (make the time spans finer)" as the firmness becomes firmer. Further, the condition may be set so as to "lower a framerate of the push pulse in the multi shot mode" and/or to "prolong the cool down time", as the firmness becomes firmer.

For example, for the five ranges illustrated in FIG. 6, obtaining conditions set with the above parameters in accordance with the firmness levels in the ranges are brought into correspondence therewith, so that correspondence information thereof is stored in the storage circuitry 44. The setting function 412 is configured to read the correspondence information corresponding to a selected range from the storage circuitry 44 and to set the read information as the obtaining conditions for the main scan.

Further, for example, the setting function 412 may be configured to input the index (the analysis result of the first shear wave) analyzed by the analyzing function 421 to a model that outputs an obtaining condition for the scan data in response to an input of the index (the analysis result of the shear wave) and to further set the obtaining condition for the scan data output by the model. For example, the setting function 412 may obtain the obtaining condition by inputting the analysis result from the analyzing function 421 to a statistical regression model or a machine learning model constructed in advance. In this situation, the statistical regression model or the machine learning model is stored in the storage circuitry 44 in advance, for instance.

In an example, the setting function 412 may calculate the adjustment parameters by using a statistical regression model presented in Expressions (1) and (2) below. Expression (1) presented below is an expression for calculating an emission interval of the tracking pulses. Expression (2) is an expression for calculating the width of the tracking pulses in the transversal direction. Further, "A" and "B" in Expression (1) and "C" in Expression (2) denote coefficients set in advance, whereas "v" in Expressions (1) and (2) denotes a propagation velocity of the shear wave.

$$\mathrm{Tracking\ Width\ =\ A \bullet v + B} \tag{1}$$

$$\mathrm{Beam\ Width\ =\ C/v} \tag{2}$$

In other words, the setting function 412 may be configured to calculate the adjustment parameters, by assigning the value of the propagation velocity calculated by the analyzing function 421 to Expressions (1) and (2). In this situation, the above expressions are merely examples, and possible embodiments are not limited to these examples. Further, although only the model related to the two adjustment parameters is presented above, it is acceptable to construct a model for calculating each of the adjustment parameters.

Further, when the machine learning model is to be constructed, it is also acceptable to construct the machine learning model so as to output obtaining conditions including all the parameters in response to an input of the analysis result. In other words, it is also acceptable to use a machine learning model that has been trained by using, as training data, the analysis result (the propagation velocities or the elasticity values) from the analyzing function 421 and the obtaining conditions including all the parameters.

As explained above, the setting function 412 is configured to set the obtaining conditions for the main scan on the basis of the analysis result of the pre-scan. Further, on the basis of the analysis result of the pre-scan, the setting function 412 is capable of setting the number of times of scan and the obtaining interval of the main scan. More specifically, when a plurality of pieces of scan data are to be consecutively obtained after the biological signal is obtained, the setting function 412 is configured to set the transmission condition of the push pulse and the transmission/reception condition of the tracking pulses for observing the shear wave, on the basis of the index. In other words, the setting function 412 is configured to set the frequency and the intervals of the push pulses and the tracking pulses on the basis of the index.

The main scan

As explained at step S106, the obtaining function 411 is configured to perform the main scan (the main SWE) on the basis of the obtaining conditions that were set. More specifically, on the basis of the obtaining conditions set by the setting function 412, the obtaining function 411 is configured to perform the main scan including the transmission of the push pulse and the transmission/reception of the tracking pulses and to thus obtain the scan data of the subject. For example, the obtaining function 411 is configured to cause the push pulse to be transmitted from the ultrasound probe 1 so that the second shear wave is caused in the biological tissue. Further, the obtaining function 411 is configured to cause the ultrasound probe 1 to transmit the tracking pulses for observing the second shear wave that occurred on the basis of the push pulse. The tracking pulses are transmitted for the purpose of observing the propagation velocities of the second shear wave generated by the push pulse at the sample points in the second measurement region R2. The obtaining function 411 is configured to generate the reflected-wave data (the scan data) from the reflected-wave signals of the tracking pulses transmitted with respect to the scanning lines in the second measurement region R2.

The evaluation information display process

As explained at step S107, the controlling function 451 is configured to cause the display 2 to display the evaluation information (e.g., an SWE image) based on the scan data obtained by the obtaining function 411. For example, the controlling function 451 may be configured to cause the display 2 to display a firmness image (an SWE image) in which colors corresponding to the elasticity values (the propagation velocities of the second shear wave) in the various positions in the second measurement region R2 to various positions (e.g., pixels) in the second measurement region R2. In that situation, for example, on the basis of the scan data obtained by performing the main scan, the controlling function 451 is configured to generate information about the propagation velocities of the second shear wave at the sample points in the second measurement region R2, as firmness distribution data. After that, the image processing function 452 is configured to generate the abovementioned firmness image (the SWE image), on the basis of the firmness distribution data. In this situation, in the present embodiment, because the color bar is also provided with a setting suitable for the measured subject on the basis of the analysis result of the first shear wave from the pre-scan, it is possible to display the firmness image that can be observed more easily.

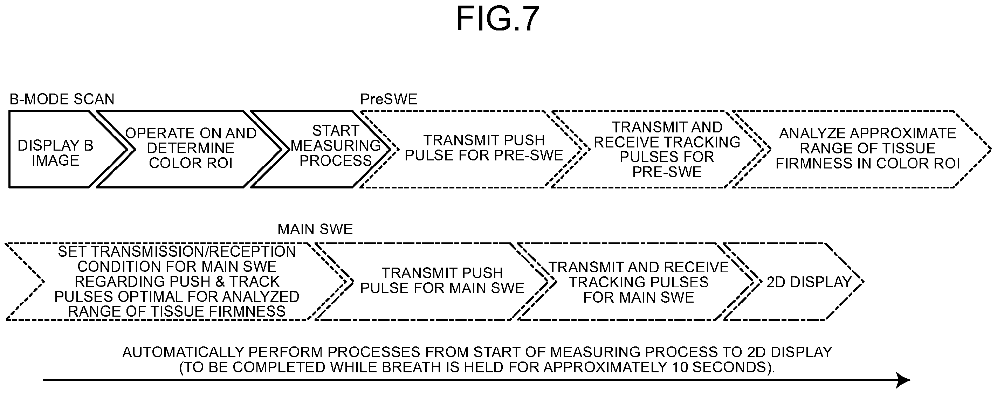

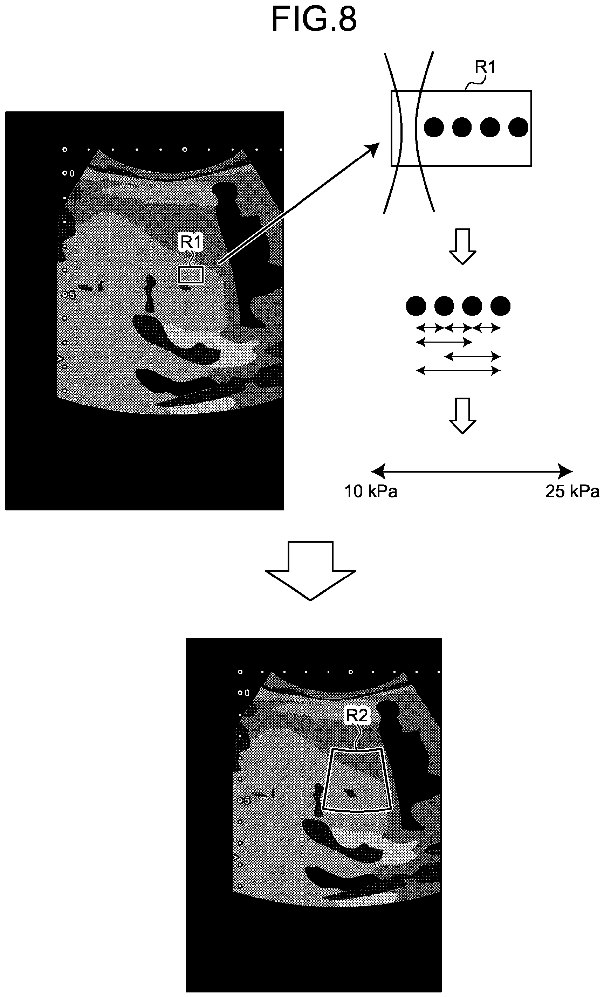

Next, application examples of the present embodiment will be explained, with reference to FIGS. 7 and 8. FIG. 7 is a drawing for explaining a sequence in a process performed by the ultrasound diagnosis apparatus 10 according to the first embodiment. FIG. 8 is a drawing for explaining processes performed by the ultrasound diagnosis apparatus 10 according to the first embodiment. FIG. 8 illustrates the processes in the "Pre SWE" and the "main SWE" in FIG. 7. In this situation, the "Pre SWE" corresponds to the pre-scan described above, whereas the "main SWE" corresponds to the main scan described above.

For example, as illustrated in FIG. 7, the ultrasound diagnosis apparatus 10 at first obtains and displays a B-mode image of the tissue serving as a measured subject of the subject, by performing a "B-mode scan", further receives an operation to set a measurement region (a color ROI) of which a firmness image is to be displayed, and determines the color ROI. In this situation, the color ROI corresponds to the region R2 described below.

Subsequently, upon receipt an instruction to start the measuring process, the ultrasound diagnosis apparatus 10 starts the "Pre SWE". In other words, the ultrasound diagnosis apparatus 10 generates a shear wave by transmitting a push pulse for the "Pre SWE" and measures the shear wave by transmitting and receiving the tracking pulses for the "Pre SWE". After that, the ultrasound diagnosis apparatus 10 analyzes an approximate range of the firmness of the tissue within the color ROI and sets transmission/reception conditions for the "main SWE" on the basis of the analysis result.

Subsequently, the ultrasound diagnosis apparatus 10 generates a shear wave by transmitting a push pulse for the "main SWE", measures the shear wave by transmitting and receiving the tracking pulses for the "main SWE", and presents a two-dimensional (2D) display of the firmness image. In this situation, the ultrasound diagnosis apparatus 10 automatically performs the processes from the start of the measuring process in the "Pre SWE" to the 2D display. It is desirable to complete the processes in this period, while the subject while the subject's breath is held for approximately 10 seconds.

In the "Pre SWE" described above, for example, the obtaining function 411 is configured to set a region R1 serving as a ROI for the "Pre SWE" in the vicinity of the region R2 (the color ROI) illustrated in FIG. 8. In other words, the region R1 (a first measurement region) is smaller than the region R2 (a second measurement region). Further, with respect to the region R1, the obtaining function 411 obtains a shear wave in a plurality of positions in the region R1, by executing a pre-scan that uses, for example, one push pulse and a number of track pulses. In this situation, the obtaining function 411 is configured to perform the pre-scan by using obtaining conditions that make it possible to calculate an approximate elasticity value in the region R1 from a wide range of firmness levels, as well as a condition that enables cool down time having a little time loss before the "main SWE (the main scan)".

The analyzing function 421 calculates a range "10 kPa to 25 kPa" of firmness from the propagation velocities of the shear wave in the plurality of positions within the region R1 obtained by performing the pre-scan. The setting function 412 sets obtaining conditions for the "main SWE" on the basis of a comparison between the calculated range of firmness and a plurality of ranges set in advance. By using the obtaining conditions set by the setting function 412, the obtaining function 411 performs the main scan on the region R2. By using a result of the main scan obtained by the obtaining function 411, the controlling function 451 causes a firmness image to be displayed in which colors corresponding to the propagation velocities of the shear wave are assigned to various positions in the region R2.

As explained above, the ultrasound diagnosis apparatus 10 according to the present embodiment is capable of setting the obtaining conditions for the main scan, on the basis of the analysis result of the pre-scan and is also capable of saving the obtaining conditions that were set. In that situation, for example, the storage circuitry 44 is configured to store therein the obtaining conditions for the scan data. The obtaining function 411 is capable of reading the obtaining conditions for the scan data from the storage circuitry 44 and obtaining scan data on the basis of the read obtaining conditions.

For example, in SWE, there may be some situations where the main scan is performed multiple times on the same subject. In those situations, the obtaining conditions that were set in the first main scan are saved into the storage circuitry 44 in correspondence with the subject so that, when the next main scan is to be performed, the obtaining function 411 performs the main scan by reading the saved obtaining conditions. With this configuration, it is possible to shorten the examination time.

As explained above, according to the first embodiment, the obtaining function 411 is configured to obtain the scan data by transmitting and receiving the ultrasound wave for observing the shear wave in the subject. Before the scan data is obtained, the obtaining function 411 is configured to obtain the biological signal of the subject. The analyzing function 421 is configured to calculate the index for determining the obtaining conditions for the scan data, on the basis of the biological signal. On the basis of the index, the setting function 412 is configured to set the obtaining conditions for the scan data. Consequently, the ultrasound diagnosis apparatus 10 according to the first embodiment is able to set the obtaining conditions for the scan data, on the basis of the biological signal of the subject, and thus makes it possible to carry out the measuring process by using the conditions suitable for the measured subject.

Further, according to the first embodiment, as the biological signal, the obtaining function 411 is configured to obtain the shear wave obtained by transmitting the push pulse and transmitting and receiving the tracking pulses. Consequently, the ultrasound diagnosis apparatus 10 according to the first embodiment makes it possible to carry out the SWE by using the obtaining conditions suitable for the tissue serving as the measured subject.

Further, according to the first embodiment, the obtaining function 411 is configured to perform the transmission of the push pulse and the transmission/reception of the tracking pulses, by using the obtaining conditions with which it is possible to obtain the shear waves propagating in the tissues having mutually-different tissue properties. Consequently, the ultrasound diagnosis apparatus 10 according to the first embodiment is able to obtain the approximate elasticity values with respect to the tissues in various states and thus makes it possible to carry out SWE while using the obtaining conditions suitable for the tissues in the various states.

Furthermore, according to the first embodiment, the analyzing function 421 is configured to calculate the level of the elasticity value in the tissue of the subject, on the basis of the shear wave. Consequently, the ultrasound diagnosis apparatus 10 according to the first embodiment is able to obtain the approximate elasticity value and thus makes it possible to easily obtain the appropriate obtaining conditions.

In addition, according to the first embodiment, the setting function 412 is configured, on the basis of the index, to select the obtaining conditions for the scan data from among the plurality of obtaining conditions set in advance. Further, the setting function 412 is configured to obtain the obtaining conditions for the scan data, by inputting the index to the model that outputs the obtaining conditions for the scan data in response to an input of the index. Consequently, the ultrasound diagnosis apparatus 10 according to the first embodiment makes it possible to easily obtain the appropriate obtaining conditions.

Further, according to the first embodiment, on the basis of the index, the setting function 412 is configured to set at least one selected from between: the time period from when the biological signal is obtained to when the obtainment of the scan data is started; and the time period until the obtainment of the next scan data is started after the scan data is obtained. Consequently, the ultrasound diagnosis apparatus 10 according to the first embodiment makes it possible to carry out the SWE with the appropriate cool down time.

Further, according to the first embodiment, the controlling function 451 is configured to control the display 2 so as to display the index. Consequently, the ultrasound diagnosis apparatus 10 according to the first embodiment is able to present the operator with the index (e.g., the elasticity value) obtained by performing the pre-scan.

Further, according to the first embodiment, when the index does not satisfy the reference level, the controlling function 451 is configured to exercise control so that the alert information related to the index is displayed. Consequently, the ultrasound diagnosis apparatus 10 according to the first embodiment is able to notify the operator when the analysis of the pre-scan is not appropriate.

Further, according to the first embodiment, when a plurality of pieces of scan data are to be consecutively obtained after the biological signal is obtained, the setting function 412 is configured to set the transmission condition of the push pulse and the transmission/reception condition of the tracking pulses for observing the shear wave, on the basis of the index. The obtaining function 411 is configured to obtain the plurality of pieces of scan data by using the transmission condition of the push pulse and the transmission/reception condition of the tracking pulses that have been set. Consequently, even when the plurality of pieces of scan data are obtained in the main scan, the ultrasound diagnosis apparatus 10 according to the first embodiment makes it possible to obtain the appropriate scan data.

Further, according to the first embodiment, as the obtaining conditions for the scan data, the setting function 412 is configured to set at least one selected from among: the transmission condition of the push pulse and the transmission/reception condition of the tracking pulses for observing the shear wave; the condition related to the display based on the scan data; and the condition related to the analysis based on the scan data. Consequently, the ultrasound diagnosis apparatus 10 according to the first embodiment makes it possible to appropriately set the various conditions related to the main scan.

Further, according to the first embodiment, the storage circuitry 44 is configured to store therein the obtaining conditions for the scan data. The obtaining function 411 is configured to read the obtaining conditions for the scan data from the storage circuitry 44 and to obtain the scan data on the basis of the read obtaining conditions. Consequently, the ultrasound diagnosis apparatus 10 according to the first embodiment is able to omit the pre-scan and thus makes it possible to shorten the examination time.

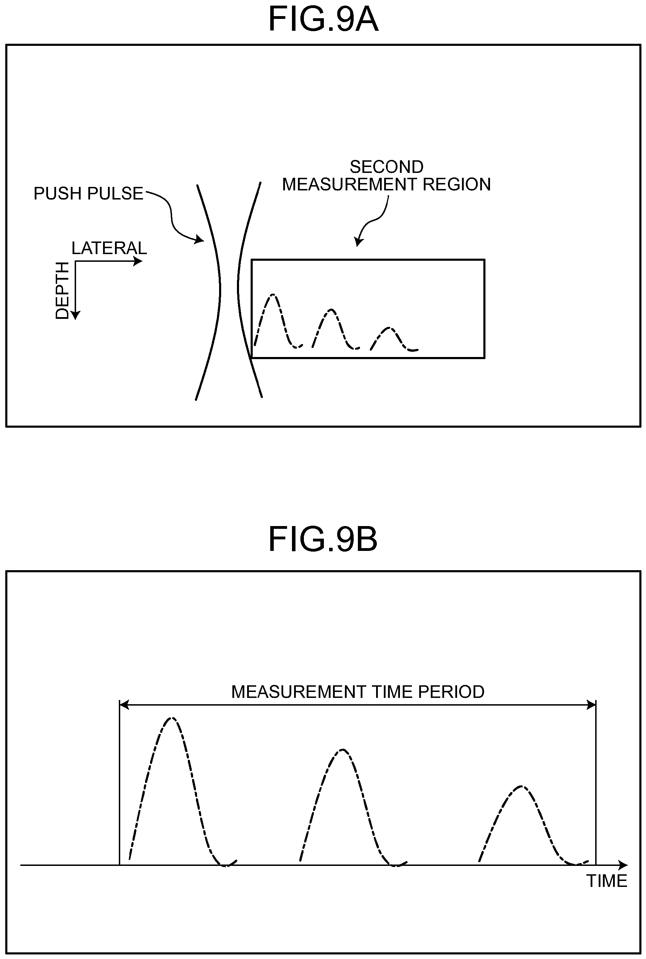

Further, according to the first embodiment, on the basis of the analysis result of the first shear wave obtained by performing the pre-scan, the setting function 412 is configured to set the conditions including at least one of the position and the size of the second measurement region R2, as the obtaining conditions for the scan data. Consequently, as illustrated in FIG. 9A, regardless of a viscoelasticity state of the tissue serving as the measured subject, it is possible to measure the shear wave within the region (the second measurement region R2) suitable for the measured subject.

Further, according to the first embodiment, on the basis of the analysis result of the first shear wave obtained by performing the pre-scan, the setting function 412 is configured to set, as the obtaining conditions for the scan data, the conditions including at least one selected from among: a measurement start time for measuring the second shear wave at various positions in the second measurement region R2; a measurement end time for the second shear wave; and a measurement time span. With this configuration, as illustrated in FIG. 9B, it is possible to measure the shear wave within the time period (the measurement time period) suitable for the measured subject, regardless of the viscoelasticity of the tissue serving as the measured subject.

Other Embodiments

In the above embodiment, the example was explained in which the obtaining conditions are set on the basis of the single analysis result (the velocity range for the propagation velocities of the shear wave); however, possible embodiments are not limited to this example. For instance, it is also acceptable to set the obtaining conditions in a more detailed manner, on the basis of a plurality of analysis results (e.g., an analysis result based on the propagation velocities and another analysis result based on the waveform information).

Further, in the above embodiment, the example was explained in which the shear wave is used as the biological signal of the subject; however, possible embodiments are not limited to this example. It is also acceptable to use, as the biological signal, reflected-wave data obtained in a B-mode scan. In other words, it is also acceptable to estimate an elasticity value of the tissue, on the basis of the reflected-wave data in the B-mode.

Further, in the above embodiment, the example using the elasticity value as the index was explained; however, possible embodiments are not limited to this example. It is also acceptable to use a viscosity value as the index. In other words, viscosity of the tissue may be analyzed on the basis of the shear wave. In that situation, the controlling function 451 may be configured to cause the display 2 to display evaluation information (e.g., a Shear Wave Dispersion (SWD) image, a viscosity image, etc.) based on the scan data obtained by the obtaining function 411. For example, the controlling function 451 may be configured to cause the display 2 to display a firmness image (an SWD image) in which colors corresponding to viscosity values (viscosity coefficients, dispersion values (dispersion slope values) of the propagation velocities with respect to frequency components) in various positions within the second measurement region R2 are assigned to various positions (e.g., pixels) in the second measurement region R2.

Further, in the above embodiment, as illustrated in FIG. 8, the example was explained in which the first measurement region R1 set in the pre-scan is smaller than the second measurement region R2 set in the main scan; however, the first measurement region R1 may be equal to the second measurement region R2 or may be larger than the second measurement region R2.

Further, in the above embodiment, as illustrated in FIG. 6, only the example was explained in which the range "10 kPa to 30 kPa" corresponding to the statistical value (e.g., the average value, the median, the range, etc.) of the elasticity values in the first measurement region R1 measured in the pre-scan was selected from among the plurality of ranges; however, it is also acceptable to cause the display 2 to display information related to the range selection during the execution of the pre-scan and/or the main scan.

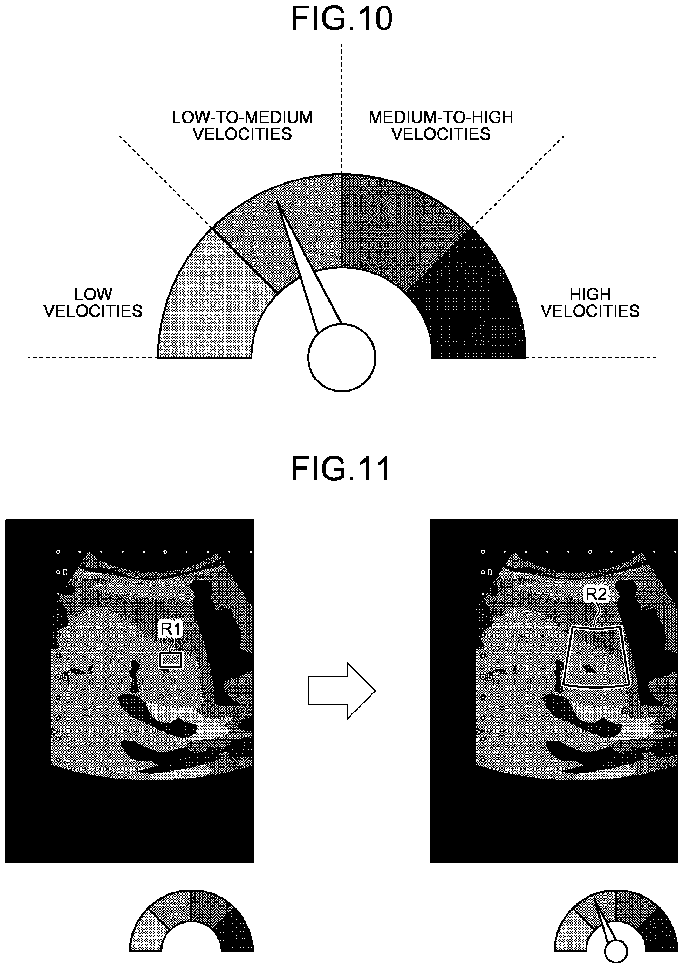

For example, the controlling function 451 may be configured to display an indicator indicating a position corresponding to the obtaining conditions set by the setting function 412, within a range where it is possible to set the obtaining conditions for the scan data. More specifically, as illustrated in FIG. 10, it is acceptable to use an indicator indicating the obtaining conditions for the scan data for measuring the propagation velocities of the shear wave corresponding to the mutually-different viscoelasticity states of the tissue. The controlling function 451 may be configured to cause the display 2 to display the indicator indicating the obtaining conditions for the scan data (for low velocities, for low-to-medium velocities, for medium-to-high velocities, and for high velocities) respectively suitable for the ranges ranging from a firmer tissue to a softer tissue.

Further, the controlling function 451 may be configured to cause an ultrasound image (e.g., a SWE image, a SWD image, etc.) and the indicator to be displayed side by side, the ultrasound image being based on the scan data (the scan data from the main scan) of the subject obtained by the obtaining function 411. For example, as illustrated in FIG. 11, during the execution of the pre-scan and the main scan, the controlling function 451 may be configured to cause the display 2 to display an indicator corresponding to each of the scans. The controlling function 451 may display the indicator before the ultrasound image (e.g., the SWE image, the SWD image, etc.) based on the scan data of the subject obtained by the obtaining function 411 is displayed, in accordance with the obtaining conditions for the scan data having been set by the setting function 412. With this configuration, the operator is able to check the obtaining conditions for the scan data (one of the conditions for low velocities, for low-to-medium velocities, for medium-to-high velocities, and for high velocities, illustrated in FIG. 10) indicated by the indicator, before the ultrasound image based on the scan data of the main scan is displayed on the display 2.

Further, in the above embodiment, the example was explained in which the setting function 412 is configured to set the obtaining conditions for the scan data, on the basis of the analysis result of the first shear wave that has propagated in the first measurement region as a result of the pre-scan; however, in other examples, obtaining conditions for the scan data may be set on the basis of at least one of B-mode data and Doppler data of the subject, and the analysis result of the first shear wave. With this configuration, it is possible to set the obtaining conditions for the scan data reflecting, in addition to the analysis result of the first shear wave, a form of the tissue of the subject expressed by the B-mode data and a blood flow state of the subject expressed by the Doppler data.

Further, the example was explained in which, on the basis of the analysis result of the first shear wave that has propagated in the first measurement region R1 as a result of the pre-scan, the setting function 412 is configured to set the obtaining conditions for the scan data for measuring the second shear wave generated in the second measurement region R2 by performing the main scan; however, it is also acceptable to set obtaining conditions for the scan data related to at least one of B-mode data and Doppler data of the subject on the basis of the analysis result of the first shear wave. With this configuration, it is possible to newly obtain the scan data related to at least one of the B-mode data and the Doppler data of the subject, while taking into consideration a state of a tissue property (viscoelasticity) of the subject.

Further, for example, because diffuse liver diseases (hepatitis, liver cirrhosis, fatty liver, etc.) are diseases in which a tissue property of the entire liver changes, a shear wave uniformly propagates in the liver. However, when the liver has other structures (blood vessels, a cyst) therein, because a shear wave non-uniformly propagates, reliability of the analysis result of the shear wave measured in such parts may be low. To cope with this situation, the analyzing function 421 may be configured to obtain, as an analysis result of the shear wave, a reliability index value indicating reliability of the propagation of the first shear wave in various positions within the first measurement region R1. For example, the analyzing function 421 may be configured to compare a parameter (elasticity index values (propagation velocities or the like)) based on the first shear wave calculated in the various positions within the first measurement region R1, with the parameter in the surroundings and to thereby obtain, as the reliability index value, a value (e.g., a difference value, a standard deviation, etc.) indicating whether or not the parameter is non-uniform in comparison to the surroundings. Further, in that situation, the setting function 412 may set obtaining conditions for the scan data on the basis of the reliability index values in various positions in the first measurement region R1. For example, the setting function 412 may be configured to identify certain positions in the first measurement region R1 where the reliability index value is smaller (i.e., where the reliability is lower) than a threshold value and to further set the obtaining conditions for the scan data in the main scan (the main SWE), on the basis of an analysis result of the first shear wave obtained from the part excluding such positions.

Further, the analyzing function 421 may obtain a reliability index value indicating reliability of the scan for the subject, on the basis of the analysis result of the shear wave that has propagated in the first measurement region R1 and at least one of B-mode data and Doppler data of the subject. In other words, the reliability index value indicating the reliability of the scan for the subject may be calculated by comprehensively analyzing, in addition to the analysis result of the first shear wave, a form of the tissue of the subject expressed by the B-mode data and a blood flow state of the subject expressed by the Doppler data. For example, the reliability index value is an index value identifying whether or not the shear wave may become non-uniform due to factors such as a structure like a blood vessel or a cyst, a region where the shear wave is weak, a region having motion, and the like. In other words, the reliability index value may be considered as an index value indicating stability of the scan (the reliability of the scan). Further, the setting function 412 may be configured to set the obtaining conditions for the scan data on the basis of the reliability index value obtained by the analyzing function 421. In other words, the obtaining conditions for the scan data may be set so as to perform the scan in a region where the scan will have high stability and reliability, on the basis of the reliability index value obtained by comprehensively analyzing the form, the property, and the blood flow state of the tissue.

In addition, in the above description, the example was explained in which the quantity of the first measurement region R1 where the first shear wave is analyzed by the analyzing function 421 is one; however, the quantity of the first measurement region R1 may be two or more (e.g., five). The plurality of measurement regions R1 may be arranged contiguously (so as to be in contact with one another) or may be arranged separately (so as to be positioned apart from one another). In that situation, the analyzing function 421 may be configured to analyze a shear wave that has propagated in each of a plurality of mutually-different regions of the subject, while the setting function 412 may be configured to set obtaining conditions for the scan data, on the basis of an analysis result of the shear wave corresponding to each of the plurality of mutually-different regions. In other words, scan data may be obtained by using a condition more suitable for the measured subject, on the basis of the analysis result of the shear wave in the plurality of measurement regions (the first measurement regions R1). For example, the setting function 412 may be configured to exclude certain analysis results (e.g., two analysis results) of which the difference (a deviation) from an average value of analysis results is larger than a prescribed threshold value, from among the analysis results (e.g., statistical values of the elasticity index values) of the first shear wave each obtained from a different one of the five first measurement regions R1. Accordingly, the setting function 412 may regard the three analysis results (the analysis results of which the deviation is equal to or smaller than the threshold value) that were not excluded as analysis results having a high reliability, so as to set the obtaining conditions for the scan data on the basis of the three analysis results.

Further, the term "processor" used in the above explanations denotes, for example, a Central Processing Unit (CPU), a Graphics Processing Unit (GPU), or circuitry such as an Application Specific Integrated Circuit (ASIC) or a programmable logic device (e.g., a Simple Programmable Logic Device (SPLD), a Complex Programmable Logic Device (CPLD), or a Field Programmable Gate Array (FPGA)). The processor is configured to realize the functions by reading and executing the programs saved in a memory. Instead of having the programs saved in the memory, the programs may directly be incorporated in the circuitry of one or more processors. In that situation, the one or more processors realize the functions by reading and executing the programs incorporated in the circuitry thereof. Further, the processors in the present embodiments do not each necessarily need to be structured as a single piece of circuitry. It is also acceptable to structure one processor by combining together a plurality of pieces of independent circuitry so as to realize the functions thereof.

The constituent elements of the apparatuses illustrated in the drawings in the description of the above embodiments are based on functional concepts. Thus, it is not necessarily required to physically configure the constituent elements as indicated in the drawings. In other words, specific modes of distribution and integration of the apparatuses are not limited to those illustrated in the drawings. It is acceptable to functionally or physically distribute or integrate all or a part of the apparatuses in any arbitrary units, depending on various loads and the status of use. Further, all or an arbitrary part of the processing functions performed by the apparatuses may be realized by a CPU and a program analyzed and executed by the CPU or may be realized as hardware using wired logic.

Further, it is possible to realize any of the methods explained in the above embodiments, by causing a computer such as a personal computer or a workstation to execute a program prepared in advance. The program may be distributed via a network such as the Internet. Further, the program may also be executed, as being recorded on a non-transitory computer-readable recording medium such as a hard disk, a flexible disk (FD), a Compact Disk Read-Only Memory (CD-ROM), a Magneto Optical (MO) disk, a Digital Versatile Disk (DVD), or a flash memory such as a Universal Serial Bus (USB) memory or a Secure Digital (SD) card memory and being read by a computer from the non-transitory recording medium.

As explained above, at least one aspect of the embodiments makes it possible to perform the measuring process by using the conditions suitable for the measured subject.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

**Claims**

1. An ultrasound diagnosis apparatus (10) comprising:

    an analyzing unit (421) configured to analyze a shear wave that has propagated in a subject;
    a setting unit (412) configured to set an obtaining condition for scan data on a basis of an analysis result of the shear wave; and
    an obtaining unit (411) configured to perform a scan on the subject on a basis of the obtaining condition and to obtain the scan data of the subject.

2. The ultrasound diagnosis apparatus (10) according to claim 1, wherein

    the analyzing unit (421) is configured to analyze a first shear wave that has propagated through various positions in a first measurement region of the subject, and
    on a basis of an analysis result of the first shear wave, the setting unit (412) is configured to set a transmission condition of a push pulse for generating a second shear wave in a second measurement region of the subject and a transmission/reception condition of a tracking pulse for observing the second shear wave, as the obtaining condition for the scan data.

3. The ultrasound diagnosis apparatus (10) according to claim 2, wherein the first measurement region is smaller than the second measurement region.

4. The ultrasound diagnosis apparatus (10) according to claim 2, wherein, on the basis of the analysis result of the first shear wave, the setting unit (412) is configured to set a condition including at least one of a position and a size of the second measurement region, as the obtaining condition for the scan data.

5. The ultrasound diagnosis apparatus (10) according to claim 2, wherein, on the basis of the analysis result of the first shear wave, the setting unit (412) is configured to set, as the obtaining condition for the scan data, a condition including at least one selected from among: a measurement start time for measuring the second shear wave in various positions in the second measurement region; a measurement end time for the second shear wave; and a measurement time span.

6. The ultrasound diagnosis apparatus (10) according to claim 1, wherein

    the analyzing unit (421) is configured to obtain a tissue property index value indicating a tissue property of the subject in each of various positions in the first measurement region of the subject, as the analysis result of the shear wave, and
    the setting unit (412) is configured to set the obtaining condition for the scan data, on a basis of the tissue property index value in each of the various positions in the first measurement region.

7. The ultrasound diagnosis apparatus (10) according to claim 6, wherein the tissue property index value is at least one selected from between: an elasticity index value indicating elasticity of a tissue of the subject; and a viscosity index value indicating viscosity of the tissue of the subject.

8. The ultrasound diagnosis apparatus (10) according to claim 2, wherein

    the analyzing unit (421) is configured to obtain a reliability index value indicating a reliability of a propagation of the first shear wave in each of various positions in the first measurement region, as the analysis result of the shear wave, and
    the setting unit (412) is configured to set the obtaining condition for the scan data on a basis of the reliability index value in each of the various positions in the first measurement region.

9. The ultrasound diagnosis apparatus (10) according to claim 1, wherein the setting unit (412) is configured to input the analysis result of the shear wave analyzed by the analyzing unit to a model that outputs an obtaining condition for the scan data in response to an input of the analysis result of the shear wave and to set the obtaining condition for the scan data output by the model.

10. The ultrasound diagnosis apparatus (10) according to claim 1, comprising: a controlling unit (451) configured to cause an indicator to be displayed, the indicator indicating a position of the obtaining condition set by the setting unit (412) within a range where it is possible to set the obtaining condition for the scan data.

11. The ultrasound diagnosis apparatus (10) according to claim 10, wherein the controlling unit (451) is configured to cause an ultrasound image and the indicator to be displayed side by side, the ultrasound image being based on the scan data of the subject obtained by the obtaining unit (411).

12. The ultrasound diagnosis apparatus (10) according to claim 1, wherein the setting unit (412) is configured to set the obtaining condition for the scan data, on a basis of at least one of B-mode data and Doppler data of the subject and the analysis result of the shear wave.

13. The ultrasound diagnosis apparatus (10) according to claim 1, wherein

the analyzing unit (421) is configured to analyze a shear wave that has propagated in each of a plurality of mutually-different regions of the subject, and
the setting unit (412) is configured to set the obtaining condition for the scan data, on a basis of the analysis result of the shear wave corresponding to each of the plurality of mutually-different regions.

14. A method comprising:

analyzing a shear wave that has propagated in a subject;
setting an obtaining condition for scan data on a basis of an analysis result of the shear wave;
performing a scan on the subject on a basis of the obtaining condition; and
obtaining the scan data of the subject.

15. A storage medium storing therein, in a non-transitory manner, a program that causes a computer to execute processes of:

analyzing a shear wave that has propagated in a subject;
setting an obtaining condition for scan data on a basis of an analysis result of the shear wave; and
performing a scan on the subject on a basis of the obtaining condition and obtaining the scan data of the subject.

# FIG.1

# FIG.2A

MANNER IN WHICH SHEAR WAVE PROPAGATES
IN SPACE DURING EACH ELAPSED TIME PERIOD

MEASUREMENT REGION

PUSH PULSE

LATERAL

DEPTH

# FIG.2B

MANNER IN WHICH SHEAR WAVE PROPAGATES
IN REGION WITH RESPECT TO EACH TRACKING BEAM

MEASUREMENT TIME PERIOD

TIME

# FIG.3

START

S101
PERFORM PRE-SCAN

S102
ANALYZE SHEAR WAVE

S103
IS ANALYSIS RESULT
APPROPRIATE?

NO

S104
DISPLAY ALERT
INFORMATION

YES

S105
SET OBTAINING CONDITIONS

S106
PERFORM MAIN SCAN

S107
DISPLAY EVALUATION
INFORMATION BASED ON SCAN
DATA

END

# FIG.4A

VELOCITY 1.2 m/s
VELOCITY 1.8 m/s
VELOCITY 1.4 m/s

R1

VELOCITY RANGE

1.2  1.8

# FIG.4B

R1

1.2 1.8 1.4

1.7  1.9

VELOCITY RANGE

1.2  1.9

## FIG.5A

ADJUST MEASUREMENT START
POSITION OF MAIN SCAN
MEASUREMENT START
POSITION OF PRE-SCAN

R1

TIME

## FIG.5B

1.2 μm

2.4 μs

TIME

1.2/2.4=0.5

m/s

AMPLITUDE/WAVELENGTH

## FIG.5C

TIME

WAVEFORM
OF 1.3 m/s

WAVEFORM
OF 1.0 m/s

1.3 m/s  CORRELATION

# FIG.6

ELASTIC MODULUS BEING CORRECT ANSWER : 15 kPa

0.75 kPa                                                                    200 kPa

RANGE SET    0.75 kPa       2 kPa        10 kPa        30 kPa      100 kPa    200 kPa
IN ADVANCE

RANGE MEASURED
IN PRE-SCAN

10 kPa              30 kPa

EP 4 585 162 A1

# FIG.7

B-MODE SCAN

DISPLAY B IMAGE → OPERATE ON AND DETERMINE COLOR ROI → START MEASURING PROCESS

PreSWE

TRANSMIT PUSH PULSE FOR PRE-SWE → TRANSMIT AND RECEIVE TRACKING PULSES FOR PRE-SWE → ANALYZE APPROXIMATE RANGE OF TISSUE FIRMNESS IN COLOR ROI

MAIN SWE

SET TRANSMISSION/RECEPTION CONDITION FOR MAIN SWE REGARDING PUSH & TRACK PULSES OPTIMAL FOR ANALYZED RANGE OF TISSUE FIRMNESS → TRANSMIT PUSH PULSE FOR MAIN SWE → TRANSMIT AND RECEIVE TRACKING PULSES FOR MAIN SWE → 2D DISPLAY

AUTOMATICALLY PERFORM PROCESSES FROM START OF MEASURING PROCESS TO 2D DISPLAY (TO BE COMPLETED WHILE BREATH IS HELD FOR APPROXIMATELY 10 SECONDS).

EP 4 585 162 A1

# FIG.8

10 kPa                    25 kPa

# FIG.9A

SECOND
MEASUREMENT REGION

PUSH PULSE

LATERAL

DEPTH

# FIG.9B

MEASUREMENT TIME PERIOD

TIME

# FIG.10

LOW-TO-MEDIUM
VELOCITIES

MEDIUM-TO-HIGH
VELOCITIES

LOW
VELOCITIES

HIGH
VELOCITIES

# FIG.11

Europäisches
Patentamt

European
Patent Office

Office européen
des brevets

## EUROPEAN SEARCH REPORT

**Application Number**

EP 25 15 2118

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/367223 A1 (HONJO YASUNORI [JP] ET AL) 22 December 2016 (2016-12-22) * paragraphs [0018], [0035], [0060], [0062], [0064], [0066], [0070], [0087], [0088], [0118]; figures 1,4,6,10 * | 1-15 | INV. A61B8/08 A61B8/00 G01S7/52 |
| A | US 2015/133783 A1 (TABARU MARIE [JP] ET AL) 14 May 2015 (2015-05-14) * paragraph [0065]; figures 9A, 9B * | 1-15 | |
| A | US 2018/238984 A1 (PAUL DOMINIK [DE] ET AL) 23 August 2018 (2018-08-23) * the whole document * | 1-15 | |
| A | US 2022/273266 A1 (YONEMORI KEITA [JP] ET AL) 1 September 2022 (2022-09-01) * the whole document * | 1-15 | |

**TECHNICAL FIELDS SEARCHED (IPC)**

A61B
G01S

The present search report has been drawn up for all claims

| Place of search | Date of completion of the search | Examiner |
|---|---|---|
| Munich | 12 May 2025 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 2118

This annex lists the patent family members relating to the patent documents cited in  the above-mentioned European search report.
The members are as contained in the European Patent Office EDP file on
The European Patent Office is in no way liable for these particulars which are merely given for the purpose of information.

12-05-2025

| Patent document cited in search report | Publication date | Patent family member(s) | | Publication date |
|---|---|---|---|---|
| US 2016367223 A1 | 22-12-2016 | JP | 6687336 B2 | 22-04-2020 |
| | | JP | 2017006213 A | 12-01-2017 |
| | | US | 2016367223 A1 | 22-12-2016 |
| US 2015133783 A1 | 14-05-2015 | CN | 104622502 A | 20-05-2015 |
| | | EP | 2881041 A1 | 10-06-2015 |
| | | JP | 5730978 B2 | 10-06-2015 |
| | | JP | 2015092937 A | 18-05-2015 |
| | | US | 2015133783 A1 | 14-05-2015 |
| US 2018238984 A1 | 23-08-2018 | DE | 102017202821 A1 | 23-08-2018 |
| | | US | 2018238984 A1 | 23-08-2018 |
| US 2022273266 A1 | 01-09-2022 | US | 2022273266 A1 | 01-09-2022 |
| | | WO | 2021241634 A1 | 02-12-2021 |

EPO FORM P0459

For more details about this annex : see Official Journal of the European Patent Office, No. 12/82